# NEW EUROPEAN PATENT SPECIFICATION

(11) **EP 1 068 311 B2**
(45) Date of publication and mention of the opposition decision: **09.12.2020**
(45) Mention of the grant of the patent: 27.04.2011
(21) Application number: 99910592.7
(22) Date of filing: 07.04.1999
(51) Int. Cl.: C12N 15/82, C12N 15/11

(54) **METHODS AND MEANS FOR OBTAINING MODIFIED PHENOTYPES**
VERFAHREN UND MITTEL ZUM ERHALT VON VERÄNDERTEN PHÄNOTYPEN
PROCEDES ET MOYENS D'OBTENTION DE PHENOTYPES MODIFIES

(30) Priority: 08.04.1998 US 56767; 03.08.1998 US 127735
(43) Date of publication of application: 17.01.2001
(60) Divisional application: 10183696.3
(73) Proprietor: Commonwealth Scientific and Industrial Research Organisation, Acton ACT 2601 (AU)
(72) Inventor: WATERHOUSE, Peter, Michael, Canberra, ACT 2602 (AU); WANG, Ming-Bo, Canberra, ACT 2612 (AU); GRAHAM, Michael, Wayne, St. Lucia, QLD 4067 (AU)
(74) Representative: Ernest Gutmann - Yves Plasseraud S.A.S.
(86) International application number: PCT/IB1999/000606
(87) International publication number: WO 1999/053050

(56) References cited:
- EP-A- 0 240 208
- EP-A- 0 467 349
- WO-A-92/13070
- WO-A-93/23551
- WO-A-94/11516
- WO-A-98/05770
- WO-A1-94/01550
- WO-A1-97/01952
- WO-A1-99/32619
- WO-A1-99/49029
- AU-A- 2 089 197
- US-A- 5 231 020
- US-A- 5 283 184
- US-A- 5 674 683
- US-A- 5 850 026
- BAULCOMBE D: "Mechanisms of pathogen-dereived resistance to viruses in transgenic plants" PLANT CELL, AMERICAN SOCIETY OF PLANT PHYSIOLOGISTS, ROCKVILLE, MD, US, vol. 8, no. 10, October 1996 (1996-10), pages 1833-1844, XP002095878 ISSN: 1040-4651
- WATERHOUSE ET AL: "Virus resistance and gene silencing in plants can be induced by simultaneous expression of sense and antisense RNA" PROCEEDINGS OF THE NATIONAL ACADEMY OF SCIENCES OF USA, NATIONAL ACADEMY OF SCIENCE. WASHINGTON, US, vol. 95, November 1998 (1998-11), pages 13959-13964, XP002114472 ISSN: 0027-8424
- STAM MAIKE ET AL: "Post-transcriptional silencing of chalcone synthase in Petunia by inverted transgene repeats" PLANT JOURNAL, vol. 12, no. 1, 1997, pages 63-82, XP002309298 ISSN: 0960-7412
- STOUTJESDIJK P A ET AL: "hpRNA-mediated targeting of the arabidopsis FAD2 gene gives highly efficient and stable silencing" PLANT PHYSIOLOGY, AMERICAN SOCIETY OF PLANT PHYSIOLOGISTS, ROCKVILLE, MD, US, vol. 129, August 2002 (2002-08), pages 1723-1731, XP002978197 ISSN: 0032-0889
- PROC. NATL. ACAD. SCI. U.S.A., Vol. 95, November 1998, P.M. WATERHOUSE et al., "Virus Resistance and Gene Silencing in Plants can be Induced by Simultaneous Expression of Sense and Antisense RNA", pages 13959-13964.
- CELL, Vol. 88, 21 March 1997, M. METZLAFF and M. O'DELL, "RNA-Mediated RNA Degradation and Chalcone Synthase A Silencing in Petunia", pages 845-854.
- TRENDS IN GENETICS, Vol. 14, No. 7, July 1998, M.K. MONTGOMERY and A. FIRE, "Double-Stranded RNA as a Mediator in Sequence Specific Genetic Silencing an Co-Suppression", pages 255-258.
- TRENDS IN GENETICS, Vol. 15, No. 1, January 1999, R.A. JORGENSEN et al., "Do Unintended Antisense Transcripts Contribute to Sense Co-Suppression in Plants?", pages 11-12.
- ANNALS OF BOTANY, Vol. 79, 1997, M. STAM et al., "The Silence of Genes in Transgenic Plants", pages 3-12.
- TRENDS IN BIOCHEMICAL SCIENCES, Vol. 18, No. 11, November 1993, W. NELLEN and C. LICHTENSTEIN, "What Makes an mRNA Anti-Sensitive?", pages 419-423.
- NATURE, Vol. 391, 19 February 1998, A. FIRE et al., "Potent and Specific Genetic Interference by Double-Stranded RNA in Caenorhabditis Elegans", pages 806-811.
- LEE, R C ET AL: "The C. elegans heterochronic gene lin-4 encodes small RNAs with antisense complementarity to lin-14" CELL, vol. 75, no. 3, 1993, pages 843-854, XP024246130
- HUNTER T, ET AL.: "THE CHARACTERISTICS OF INHIBITION OF PROTEIN SYNTHESIS BY DOUBLE-STRANDED RIBONUCLEIC ACID IN RETICULOCYTE LYSATES", Journal of Biological Chemistry, American Society for Biochemistry and Molecular Biology, US, vol. 250, no. 02, 25 January 1975 (1975-01-25), pages 409-417, US ISSN: 0021-9258
- Carolyn Napoli et al: "lntroduction of a Chimeric Chalcone Synthase Gene into Petunia Results in Reversible Co-Suppression of Homologous Genes Ín trans", The Plant Cell, vol. 2, 1 April 1990 (1990-04-01), pages 279-289,
- HAMILTON A J et al: "ANTISENSE GENE THAT INHIBITS SYNTHESIS OF THE HORMONE ETHYLENE IN TRANSGENIC PLANTS", NATURE, Nature Publishing Group UK, London, vol. 346, 19 July 1990 (1990-07-19), pages 284-287, London ISSN: 0028-0836, DOI: 10.1038/346284a0
- Hobbs, Shaun L.A. et al: "The effect of T-DNA copy number, position and methylation on reporter gene expression in tobacco transformants", PLANT MOLECULAR BIOLOGY, SPRINGER, DORDRECHT., NL, vol. 15, no. 6, 1 January 1990 (1990-01-01), pages 851-864, NL ISSN: 0167-4412, DOI: 10.1007/BF00039425
- INGELBRECHT I ET AL: "TRANSCRIPTIONAL INTERFERENCE IN TRANSGENIC PLANTS", GENE., ELSEVIER, AMSTERDAM., NL, vol. 109, no. 2, 1 January 1991 (1991-01-01), pages 239-242, NL ISSN: 0378-1119, DOI: 10.1016/0378-1119(91)90614-H
- Romano, Nicoletta et al: "Quelling: transient inactivation ofgene expression in Neurospora crassa by transformation with homologous sequences", Molecular Microbiology, vol. 6, no. 22, 1 November 1992 (1992-11-01), pages 3343-3353,
- Hobbs, Shaun L.A. et al: "Transgene copy number can bepositively or negatively associated with transgeneexpression", Plant Molecular Biology, vol. 21, 1 January 1993 (1993-01-01), pages 17-26,
- Leech, Mark J. et al: "Expression of myb-related genes inthe moss, physcomitrella patens", The Plant Journal, vol. 3, no. 1, 1 January 1993 (1993-01-01) , pages 51-61, & GenBank Entry No. X67051 for P.patens Pp1 & mFOLD RNA secondary structure prediction for GenBankEntry No. X67051 & GenBank Entry No. X67051 for P. patens Pp1 & GenBank Entry No. X67050 for P. patens Pp2
- MEMELINK J, ET AL: "Structure and regulation of tobacco extensin.", THE PLANT JOURNAL, BLACKWELL SCIENTIFIC PUBLICATIONS, OXFORD., GB, vol. 4, no. 6, 1 December 1993 (1993-12-01), pages 1011-1022, GB ISSN: 0960-7412 & GenBank Entry No. X71602 for N.tabacum extensin & mFOLD RNA secondary structure prediction for GenBankEntry No. X71602
- DIEFFENBACH C. W. et al: "GENERAL CONCEPTS FOR PCR PRIMER DESIGN.", PCR METHODS & APPLICATIONS., COLD SPRING HARBOR LABORATORY PRESS., US, vol. 03., 1 December 1993 (1993-12-01), pages S30-S37., US ISSN: 1054-9803
- BUREAU T E ET AL: "Stowaway: a new family of inverted repeat elements associated with the genes of both monocotyledonous and dicotyledonous plants.", The Plant Cell, American Society of Plant Biologists, US, vol. 6, no. 6, 1 June 1994 (1994-06-01), pages 907-916, XP002156106, US ISSN: 1040-4651, DOI: 10.1105/tpc.6.6.907
- van Blokland, R. et al: "Transgene-mediated suppression of chalcone synthase expression in Petunia hybrida results from an increase in RNA turnover", The Plant Journal, vol. 6, no. 6, 1 December 1994 (1994-12-01), pages 861-877,
- Falcone, Deane et al: "Identification of a Gene that Complements an Arabidopsis Mutant Deficient in Chloroplast omega-6 Desaturase Activity1", Plant Physiology, vol. 106, no. 4, 1 December 1994 (1994-12-01), pages 1453-1459,
- Kola, Ismail et al: "Microinjection of In Vitro Transcribed RNA and Antisense Oligonucleotides in Mouse Oocytes and Early Embryos to Study the Gain- and Loss-of-Function of Geness", Methods in Molecular Biology, vol. 37, 1 January 1995 (1995-01-01), pages 135-137,
- Zrenner, Rita at al: "Evidence of the crucial role of sucrose synthase for sink strength using transgenic potato plants (Solanum tuberosum L.)", The Plant Journal, vol. 7, no. 1, 1 January 1995 (1995-01-01) , pages 97-107, & GenBank Entry No U21129 for S. tuberosum sucrose synthase & mFOLD RNA secondary structure prediction for GenBank Entry No. U21129 & Detail from mFOLD RNA secondary structure prediction for S. tuberosum sucrose synthase RNA
- GUO S, KEMPHUES K J: "PAR-1, A GENE REQUIRED FOR ESTABLISHING POLARITY IN C. ELEGANS, EMBRYOS, ENCODES A PUTATIVE SER/THR KINASE THAT IS ASYMMETRICALLY DISTRIBUTED", CELL, ELSEVIER, AMSTERDAM, NL, vol. 81, 19 May 1995 (1995-05-19), pages 611-620, AMSTERDAM, NL ISSN: 0092-8674, DOI: 10.1016/0092-8674(95)90082-9
- Creissan Gary et al: "Simultaneous targeting of pea glutathione reductase and of a bacterial fusion protein to Chloroplasts and mitochondria in transgenic tobacco", The plant Journal, vol. 8, no. 2, 1 August 1995 (1995-08-01), pages 167-175,
- Blokland R. van et al: "Post-transcriptional suppresssion of chalcone synthase genes in Petunia Hybrids and the accumulation of unspliced PRE-mRNAS" In: Grierson et al (Eds): "Mechanisms and Applications of Gene Silencing", 1996, Nottingham University Press pages 57-69,
- Klaff P. et al: "RNA structure and the regulation of gene expression", Plant Molecular Biology, vol. 32, 1 October 1996 (1996-10-01), pages 89-106,
- Sijen Titia et al: "RNA-Mediated Virus Resistance: Role of Repeated Transgenes and Delineation of Targeted Regions", The plant cell, vol. 8, 1 December 1996 (1996-12-01), pages 2277-2294,
- Lewin Benjamin: "Genes VI", Oxford University Press and Cell Press, 1997, pages 101-105-106,
- Azpiroz-leehan, R. Feldmann, K.A.: "T-DNA insertion mutagenesis in Arabidopsis: going back and forth", TRENDS IN GENETICS., ELSEVIER SCIENCE PUBLISHERS B.V. AMSTERDAM., NL, vol. 13, no. 4, 1 April 1997 (1997-04-01), pages 152-156, NL ISSN: 0168-9525, DOI: 10.1016/S0168-9525(97)01094-9
- Bluma B. et al: "Identification of transposon-like elements in non-coding regions of tomato ACC oxidase genes", Mol. Gen. Genet., vol. 254, 16 April 1997 (1997-04-16), pages 297-303,
- Takahashi, Hideki et al: "Development of necrosis and activation of disease resistance in transgenic tobacco plants with severely reduced catalase levels", The Plant Journal, vol. 11, no. 5, 1 May 1997 (1997-05-01), pages 993-1005, & GenBank Entry No. U93244 for N. tabacum catalase & mFOLD RNA secondary structure prediction GenBank Entry No. U93244 & Detall from mFOLD RNA secondary structure prediction for N. tabacum catalase
- RATCLIFF F. ET AL.: "A similarity between viral defense and gene silencing in plants", Science, American Association for the Advancement of Science, US, vol. 276., 6 June 1997 (1997-06-06), pages 1558-1560., US ISSN: 0036-8075, DOI: 10.1126/science.276.5318.1558
- Palauqui, Jean-Christophe et al: "Systemic acquired silencing: transgene-specific post-transcriptional silencing is transmitted by grafting from silenced stocks to non-silenced scions", the EMBO Journal, vol. 16, no. 15, 1 August 1997 (1997-08-01), pages 4738-4745,
- Que, Qiudeng et al: "The Frequency and Degree of Cosuppression by Sense Chalcone Synthase Transgenes Are Dependent on Transgene Promoter Strength and Are Reduced by Premature Nonsense Codons in the Transgene Coding Sequence", The Plant Cell, vol. 9, August 1997 (1997-08), pages 1357-1368,
- Tanzer, M. et al: "Characterization of Post-Transcriptionally Suppressed Transgene Expression That Confers Resistance to Tobacco Etch Virus Infection in Tobacco", The Plant Cell, vol. 9, August 1997 (1997-08), pages 1411-1423,
- Selker, Eric U.: "Epigenetic phenomena in filamentous fungi: useful paradigms or repeat-induced confusion?", TIG, vol. 13, no. 8, August 1997 (1997-08), pages 296-301,
- Lee, Kathleen Y. et al: "Post-transcriptional gene silencing of ACC synthase in tomato results from cytoplasmic RNA degradation", The Plant Journal, vol. 12, no. 5, November 1997 (1997-11), pages 1127-1137,
- Cogoni, Carlo et al: "Conservation of transgene-induced post-transcriptional gene silencing in plants and fungi", trends in plant science, vol. 2, no. 11, November 1997 (1997-11), pages 438-443,
- Smyth, David R.: "Gene silencing: Cosuppression at a distance", CURRENT BIOLOGY, vol. 7, 1 December 1997 (1997-12-01), pages R793-R795,
- Wagner R. W and Sun L.: "Double-stranded RNA poses puzzle", Nature, vol. 391, 19 February 1998 (1998-02-19), pages 744-745,

## Description

### 1. Field of the Invention

The invention relates to methods for reducing the phenotypic expression of a nucleic acid sequence of interest in eucaryotic cells, particularly plant cells, by simultaneously providing the cells with chimeric genes encoding sense and anti sense RNA molecules comprising nucleotide sequences respectively homologous and complementary to at least part of the nucleotide sequence of the nucleic acid of interest. The sense and antisense RNA molecules are provided as one RNA molecule, wherein the sense and antisense RNA may be linked by a spacer nucleotide sequence and are capable of forming a double stranded RNA molecule.

In one aspect of the invention, the methods are directed towards reducing viral infection, resulting in extreme virus resistance. In another embodiment the methods are directed towards reducing the phenotypic expression of an endogenous gene in a plant cell. Also provided are plant cells comprising such RNA molecules, as well as plants consisting essentially of such plant cells.

### 2. Background of the Invention

In 1985, Sanford and Johnston proposed the concept of parasite-derived resistance. They postulated that key gene products from a parasite expressed in the host in a dysfunctional form, in excess or at a wrong developmental stage, should disrupt the function of the parasite with minimal effect on the host (Sanford & Johnston, 1985). Using the QB bacteriophage as a model, they proposed that expression, in bacteria, of the bacteriophage coat protein or modified replicase or an antisense RNA complementary to the QB genome could all give resistance. They also proposed that such approaches would be applicable, in plants, to plant viruses and particularly the use of a modified plant virus replicase. The expression of the coat protein of the plant virus, tobacco mosaic virus (TMV), in tobacco was the first practical validation of this concept for plant virus resistance. This work (Powell-Abel *et al*., 1986) showed that the expression of the TMV coat protein, from a transgene under the control of the cauliflower mosaic virus 35S promoter, conferred on the plants resistance to TMV. The same group (Powell *et al.,* 1990) showed that, generally, plants expressing higher levels of coat protein were more resistant to TMV than plants expressing low levels. Since this demonstration there have been very many examples of plants transformed with virus coat protein genes showing resistance (Table 1). There have also been a number of reports of plant virus resistance in plants expressing wild-type replicase (Braun and Hemenway, 1992, Brederode *et al.,* 1995), truncated replicase (Carr *et al.* 1992), modified replicase (Longstaff *et al.* 1993), or antisense viral RNA (Kawchuck *et al.* 1991).

In 1992, Dougherty and colleagues were using different forms of the coat protein gene of tobacco etch virus (TEV) and discovered that some plants containing untranslatable "sense" coat protein genes and antisense coat protein genes showed extreme resistance (ER) to the virus (Lindbo & Dougherty, 1992 a,b). This resistance was functional at the whole plant level and at the single cell level. TEV was unable to replicate in protoplasts derived from ER plants but replicated to high levels in protoplasts from non-transgenic tobacco. Dougherty et al. concluded that the mechanism creating the extreme resistance for the untranslatable sense construct was not the same as the often reported coat protein-mediated strategy. They proposed that the mRNA of the untranslatable sense construct was hybridizing with the minus sense genome of the virus and interfering with the procession of replication complexes on the minus strand. They suggested that the use of viral sequence that could form intramolecular pairing should be avoided as this would interfere with their ability to hybridize to the target viral RNA.

**Table 1: Plant species that have been genetically engineered for virus resistance (from Rebecca Grumet, Hort Science 30[3] 1995)**

| **Species** | **Viruses** |
|---|---|
| **Tobacco** (*Nicotiana tabacum* L.) | AIMV, ArMV, CMV, PVX, PVY, TEV, TGMV, TMV, TRV, TSV, TSWV |
| **Other Nicotiana spp.** (*N. debneyii, N. benthamiana, N. clevelandii*) | ACMV, BYMV, CyMV, CyRSV, BCTV, PEBV, PPV, PVS, WMV |
| **Potato** (*Solanum tuberusom* L.) | PI, RV, PVY |
| **Tomato** (*Lycopersicon esculentum* L.) | AIMV, CMV, TMV, TYLCV |
| **Cucumber** (*Cucumis sativus* L.) | CMV |
| **Melon** (*Cucumis melo* L.) | CMV, ZYMV |
| **Alfalfa** (*Medicago sativa* L.) | AIMV |
| **Papaya** (*Carica papaya* L.) | PRSV |
| **Corn** (*Zea mays* L.) | MDMV |
| **Rice** (*Oryza sativa* L.) | RSV |
| **Rapeseed** (*Brassica napus* L.) | TYMV |

The Dougherty group expanded their investigations to plants containing untranslatable sense potato virus Y (PVY) coat protein genes. They obtained results similar to those with TEV and showed that the plants with ER had high transgene copy number, highly active transcription of the transgenes, and low levels of steady state mRNA from the PVY transgene (Lindbo *et al.* 1993, Smith *et al.* 1994). The following model for this mechanism of the resistance was proposed: the high level of transcription of the viral transgene triggers a cytoplasmic based, post transcriptional cellular surveillance system that targets specific RNAs for elimination. As the transgene encodes a transcript comprising viral sequences the mechanism not only degrades the transgene mRNA but also the same sequences in the viral genomic RNA. A key point in this model is the need for a high level of transcription of the transgene provided by high copy number (3-8; Goodwin et al. 1996). Alternatively, the RNA threshold required to trigger the mechanism can be reached by a more modest transcription level aided by the viral RNA from replication in early infection. This gives rise to a "recovery phenotype" where the plant is initially infected and shows symptoms but then produces new growth without virus symptoms and which are extremely resistant to infection.

This proposal was substantiated by the findings that gene silencing of non-viral transgenes could also be due to a post transcriptional mechanism (Ingelbrecht et al. 1994; de Carvalho Niebel et al. 1995) operating at an RNA level.

A link between non-viral gene silencing and this pathogen derived resistance was provided by inoculating transgenic plants, in which a GUS transgene was known to be silenced by a post transcriptional mechanism, with a virus containing GUS sequences (English et al. 1996). In this situation the plants were extremely resistant to the viral infection. However, the same plants were susceptible to the virus if they contained no GUS sequences.

The degree of viral resistance is not always directly related to the level of viral transgene transcription (Mueller et al. 1995; English et al. 1996) suggesting that there may be an alternative mechanism of inducing the resistance. To accommodate these discrepancies, an alternative model has been proposed in which the crucial factor affecting the resistance is not the level but the quality of the transgene mRNA (English et al. 1996). According to this model, the transgene can only induce resistance (or gene silencing) if it is transcribed to produce "aberrant" RNA. There have been many examples of post-transcriptional gene silencing and methylation of the transgene (Hobbs et al. 1990; Ingelbrecht et al. 1994) and methylation of the transgene has also been found to be associated in some cases of extreme viral resistance (Smith et al. 1994, English 1996). In the proposed model, methylation of the transgene leads to the production of aberrant RNAs which induce the cytoplasmic RNA surveillance system. Baulcombe and English have suggested that this method of induction may be the same as that found for the silencing of met2 in A.immersus. In this system transcription of the met2 RNA was terminated in the methylated regions of the endogenous gene thus producing aberrant truncated RNAs. It was suggested that the methylation was a consequence of ectopic interaction between the transgene and a homologous region of a corresponding region of the endogenous gene (Barry et al. 1993). The presence of multiple transgenes would create an increased likelihood of ectopic pairing and is therefore consistent with the correlation between high copy number and extreme viral resistance (Mueller et al., 1995; Goodwin et al. 1996; Pang et al., 1996).

This whole area has been reviewed recently (e.g. Baulcombe (1996) and Stam et al. (1997)) and several models were presented. All models call for a high degree of sequence specificity because the resistance is very (strain) specific and therefore invoke base pairing interaction with an RNA produced from the transgene. One explanation for the induction of the virus resistance or gene silencing with sense transgenes is that the plant's RNA dependent RNA polymerase generates complementary RNAs using the transgene mRNA as a template (Schiebel et al. 1993a,b). This hypothetical complementary RNA (cRNA) has not been detected (Baulcombe 1996) but it is expected that the cRNAs will be small and heterodisperse RNAs rather than full complements (Schiebel 1993ab, Baulcombe 1996) and therefore difficult to detect.

The possible methods of action of the cRNA in mediating the virus resistance or gene silencing (as proposed by Baulcombe, 1996) are :
1: The cRNA hybridizes with transgene mRNA or viral RNA and the duplex becomes a target for dsRNases.;
2: The cRNA hybridizes with target RNA to form a duplex which can arrest translation and consequently have an indirect effect on stability (Green, 1993);
3: The duplex formed between the cRNA and viral RNA causes hybrid arrest of translation of co-factors required for viral replication; and
4. The hybridization of the cRNA affects intra-molecular base pairing required for viral replication.

The current models for virus resistance or gene silencing thus involve the induction of a cytoplasmic surveillance system by either high levels of transgene transcription or by the production of aberrant single stranded mRNA. Once the system is triggered, RNA dependent RNA polymerase makes cRNA from the transgene mRNA. These cRNAs hybridize to the target RNA either directly affecting its translatability or stability, or marking the RNA for degradation.

US 5,190,131 and EP 0 467 349 A1 describe methods and means to regulate or inhibit gene expression in a cell by incorporating into or associating with the genetic material of the cell a non-native nucleic acid sequence which is transcribed to produce an mRNA which is complementary to and capable of binding to the mRNA produced by the genetic material of that cell.

EP 0 223 399 A1 describes methods to effect useful somatic changes in plants by causing the transcription in the plant cells of negative RNA strands which are substantially complementary to a target RNA strand. The target RNA strand can be a mRNA transcript created in gene expression, a viral RNA, or other RNA present in the plant cells. The negative RNA strand is complementary to at least a portion of the target RNA strand to inhibit its activity in vivo.

EP 0 240 208 describes a method to regulate expression of genes encoded for in plant cell genomes, achieved by integration of a gene under the transcriptional control of a promoter which is functional in the host and in which the transcribed strand of DNA is complementary to the strand of DNA that is transcribed from the endogenous gene(s) one wishes to regulate.

EP 0 647 715 A1 and US patents 5, 034,323, 5,231,020 and 5,283,184 describe methods and means for producing plants exhibiting desired phenotypic traits, by selecting transgenotes that comprise a DNA segment operably linked to a promoter, wherein transcription products of the segment are substantially homologous to corresponding transcripts of endogenous genes, particularly endogenous flavonoid biosynthetic pathway genes.

WO 93/23551 describes methods and means for the inhibition of two or more target genes, which comprise introducing into the plant a single control gene which has distinct DNA regions homologous to each of the target genes and a promoter operative in plants adapted to transcribe from such distinct regions RNA that inhibits expression of each of the target genes.

WO92/13070 describes a method for the regulation of nucleic acid translation, featuring a responsive RNA molecule which encodes a polypeptide and further includes a regulatory domain, a substrate region and a ribosome recognition sequence. This responsive RNA molecule has an inhibitor region in the regulatory domain, which regulatory domain is complementary to both a substrate region of the responsive RNA molecule and to an anti-inhibitor region of a signal nucleic acid such that, in the absence of the signal nucleic acid, the inhibitor and substrate regions form a base-paired domain the formation of which reduced the level of translation of one of the protein-coding regions in the responsive RNA molecule compared to the level of translation of that one protein-coding region observed in the presence of the signal nucleic acid.

Metzlaff et al., 1997 describe a model for the RNA-mediated RNA degradation and chalcone synthase A silencing in Petunia, involving cycles of RNA-RNA pairing between complementary sequences followed by endonucleolytic RNA cleavages to describe how RNA degradation is likely to be promoted. Fire et al., 1998 describe specific genetic interference by experimental introduction of double-stranded RNA in Caenorhabditis elegans. The importance of these findings for functional genomics is discussed (Wagner and Sun, 1998).

Que et al., 1998 describe distinct patterns of pigment suppression which are produced by allelic sense and antisense chalcone synthase transgenes in petunia flowers and have also analyzed flower color patterns in plants heterozygous for sense and antisense chalcone synthase alleles.

WO 98/05770 discloses antisense RNA with special secondary structures which may be used to inhibit gene expression.

WO 94/18337 discloses transformed plants which have increased or decreased linolenic acids as well as plants which express a linoleic acid desaturase.

US 5,850,026 discloses an endogenous oil from Brassica seeds that contains, after crushing and extracting, greater than 86% oleic acid and less than 2.5% 〈-linolenic acid. The oil also contains less than 7% linoleic acid. The Brassica seeds are produced by plants that contain seed-specific inhibition of microsomal oleate desaturase and microsomal linoleate desaturase gene expression, wherein the inhibition can be created by cosuppression or antisense technology.

US 5,638,637 discloses vegetable oil from rapeseeds and rapeseed producing the same, the vegetable oil having an unusually high oleic acid content of 80% to 90% by weight based on total fatty acid content.

### SUMMARY OF THE INVENTION

The present invention provides methods for reducing the phenotypic expression of a nucleic acid of interest, which can be expressed in a eucaryotic cell, particularly for reducing the phenotypic expression of a gene, particularly a endogenous gene or a foreign transgene, integrated in the genome of a eucaryotic cell or for reducing the phenotypic expression of nucleic acid of interest which is comprised in the genome of an infecting virus, comprising the step of introducing into said cell, preferably integrating, in the nuclear genome of the eucaryotic cell, a chimeric DNA comprising a promoter, capable of being expressed in that eucaryotic cell, and optionally a DNA region involved in transcription termination and polyadenylation and in between a DNA region, which when transcribed, yields an RNA molecule with a nucleotide sequence comprising a sense nucleotide sequence including at least 15 consecutive nucleotides, particularly at least about 550 consecutive nucleotides, having 100% sequence identity with at least part of a coding region of the nucleic acid of interest, and an antisense nucleotide sequence including at least 15 consecutive nucleotides, having 100% sequence identity with the 15 nucleotide stretch of the complement of the sense nucleotide sequence, wherein the RNA is capable of forming an artificial hairpin RNA structure with a double stranded RNA stem by base-pairing between the regions with sense and antisense nucleotide sequence such that at least the 15 consecutive nucleotides of the sense sequence base pair with the 15 consecutive nucleotides of the antisense sequence, resulting in an artificial hairpin structure, wherein said nucleic acid of interest is a gene integrated in the genome of said eukaryotic cell, and wherein said method is not a method of treatment of the human or animal body by surgery or therapy, or a diagnostic method practised on the human or animal body.

The invention also provides a eucaryotic cell, comprising a nucleic acid of interest which can be phenotypically expressed, further comprising a chimeric DNA molecule comprising a promoter, capable of being expressed in that eucaryotic cell, a DNA region, which when transcribed, yields an RNA molecule with at least one RNA region with a nucleotide sequence comprising a sense nucleotide sequence including at least 15 consecutive nucleotides having 100% sequence identity with at least part of a coding region of the nucleic acid of interest and an antisense nucleotide sequence including at least 15 consecutive nucleotides, having 100% sequence identity with the complement of the at least 15 consecutive nucleotides of the sense nucleotide sequence wherein the RNA molecule is capable of forming an artificial hairpin RNA structure with a double-stranded RNA stem a by basepairing between the regions with sense and antisense nucleotide sequence such that at least said 15 consecutive nucleotides of the sense sequence base pair with said 15 consecutive nucleotides of the antisense sequence, said nucleic acid of interest is a gene integrated in the genome of said eukaryotic cell, and preferably a DNA region involved in transcription termination and polyadenylation, wherein the phenotypic expression of the nucleic acid of interest is significantly reduced.

With the foregoing and other objects, advantages and features of the invention that will become hereinafter apparent, the nature of the invention may be more clearly understood by reference to the following detailed description of the preferred embodiments of the invention and to the appended claims.

### BRIEF DESCRIPTION OF THE DRAWINGS

**Figure 1** represents schematically the different sense and antisense constructs, as well as the so-called CoP (complementary pair) constructs used for obtaining virus resistance (Fig 1 B) or for reducing the phenotypic expression of a transgenic Gus gene (Fig 1 A).
**Figure 2A** represents schematically the so-called panhandle construct or CoP constructs used for reducing the phenotypic expression of a Δ12 desaturase gene in Arabidopsis (Nos Pro: nopaline synthase gene promoter; nptll neomycin phosphotransferase coding region; Nos term: nopaline syntase gene terminator; FP1: truncated seed specific napin promoter; 480 bp: 5' end of the Fad2 gene of Arabidopsis thaliana in sense orientation; 623 bp: spacer; 480 bp: 5' end of the Fad2 gene of Arabidopsis thaliana in antisense orientation.
**Figure 2B** represents schematically a common cosuppression construct for for reducing the phenotypic expression of at Δ12 desaturase gene in Arabidopsis thaliana.

### DETAILED DESCRIPTION OF THE PREFERRED EMBODIMENTS OF THE INVENTION

Herein disclosed is a eucaryotic cell, particularly a plant cell with an RNA molecule which comprises a hairpin structure including a determined sense part and a determined antisense part. Potentially, an RNA molecule is capable of forming several secondary structures, some of which may contain the desired hairpin. It is expected that the real secondary structure of the RNA in the cell, will have the lowest free energy. In accordance with the invention, the RNA molecule to be produced in the cell is designed in such a way that at least in its lowest free energy state, which it can assume within that cell, it will comprise the desired hairpin.

As used herein "hairpin RNA" refers to any self-annealing double stranded RNA molecule. In its simplest representation, a hairpin RNA consists of a double stranded stem made up by the annealing RNA strands, connected by a single stranded RNA loop, and is also referred to as a "pan-handle RNA". However, the term "hairpin RNA" is also intended to encompass more complicated secondary RNA structures comprising self-annealing double stranded RNA sequences, but also internal bulges and loops. The specific secondary structure adapted will be determined by the free energy of the RNA molecule, and can be predicted for different situations using appropriate software such as FOLDRNA (Zuker and Stiegler, 1981).

As used herein, "sequence identity" with regard to nucleotide sequences (DNA or RNA), refers to the number of positions with identical nucleotides divided by the number of nucleotides in the shorter of the two sequences. The alignment of the two nucleotide sequences is performed by the Wilbur and Lipmann algorithm (Wilbur and Lipmann, 1983) using a window-size of 20 nucleotides, a word length of 4 nucleotides, and a gap penalty of 4. Computer-assisted analysis and interpretation of sequence data, including sequence alignment as described above, can, e.g., be conveniently performed using the programs of the Intelligenetics^{™} Suite (Intelligenetics Inc., CA). Sequences are indicated as "essentially similar" when such sequence have a sequence identity of at least about 75%, particularly at least about 80 %, more particularly at least about 85%, quite particularly about 90%, especially about 95%, more especially about 100%, quite especially are identical. It is clear than when RNA sequences are said to be essentially similar or have a certain degree of sequence identity with DNA sequences, thymine (T) in the DNA sequence is considered equal to uracil (U) in the RNA sequence.

As used herein, the term "plant-expressible promoter" means a DNA sequence which is capable of controlling (initiating) transcription in a plant cell. This includes any promoter of plant origin, but also any promoter of non-plant origin which is capable of directing transcription in a plant cell, i.e., certain promoters of viral or bacterial origin such as the CaMV35S, the subterranean clover virus promoter No 4 or No 7, or T-DNA gene promoters.

The term "expression of a gene" refers to the process wherein a DNA region which is operably linked to appropriate regulatory regions, particularly to a promoter, is transcribed into an RNA which is biologically active i.e., which is either capable of interaction with another nucleic acid or which is capable of being translated into a polypeptide or protein. A gene is said to encode an RNA when the end product of the expression of the gene is biologically active RNA, such as e.g. an antisense RNA, a ribozyme or a replicative intermediate. A gene is said to encode a protein when the end product of the expression of the gene is a protein or polypeptide.

A nucleic acid of interest is "capable of being expressed", when said nucleic acid, when introduced in a suitable host cell, particularly in a plant cell, can be transcribed (or replicated) to yield an RNA, and/or translated to yield a polypeptide or protein in that host cell.

The term "gene" means any DNA fragment comprising a DNA region (the "transcribed DNA region") that is transcribed into a RNA molecule (e.g., an mRNA) in a cell operably linked to suitable regulatory regions, e.g., a plant-expressible promoter. A gene may thus comprise several operably linked DNA fragments such as a promoter, a 5' leader sequence, a coding region, and a 3' region comprising a polyadenylation site. A plant gene endogenous to a particular plant species (endogenous plant gene) is a gene which is naturally found in that plant species or which can be introduced in that plant species by conventional breeding. A chimeric gene is any gene which is not normally found in a plant species or, alternatively, any gene in which the promoter is not associated in nature with part or all of the transcribed DNA region or with at least one other regulatory region of the gene.

As used herein, "phenotypic expression of a nucleic acid of interest" refers to any quantitative trait associated with the molecular expression of a nucleic acid in a host cell and may thus include the quantity of RNA molecules transcribed or replicated, the quantity of post-transcriptionally modified RNA molecules, the quantity of translated peptides or proteins, the activity of such peptides or proteins.

A "phenotypic trait" associated with the phenotypic expression of a nucleic acid of interest refers to any quantitative or qualitative trait, including the trait mentioned, as well as the direct or indirect effect mediated upon the cell, or the organism containing that cell, by the presence of the RNA molecules, peptide or protein, or posttranslationally modified peptide or protein. The mere presence of a nucleic acid in a host cell, is not considered a phenotypic expression or a phenotypic trait of that nucleic acid, even though it can be quantitatively or qualitatively traced. Examples of direct or indirect effects mediated on cells or organisms are, e.g., agronomically or industrial useful traits, such as resistance to a pest or disease; higher or modified oil content etc.

As used herein, "reduction of phenotypic expression" refers to the comparison of the phenotypic expression of the nucleic acid of interest to the eucaryotic cell in the presence of the chimeric genes of the invention, to the phenotypic expression of the nucleic acid of interest in the absence of the chimeric genes of the invention. The phenotypic expression in the presence of the chimeric RNA should thus be lower than the phenotypic expression in absence thereof, preferably be only about 25%, particularly only about 10%, more particularly only about 5% of the phenotypic expression in absence of the chimeric RNA, especially the phenotypic expression should be completely inhibited for all practical purposes by the presence of the chimeric RNA or the chimeric gene encoding such an RNA.

A reduction of phenotypic expression of a nucleic acid where the phenotype is a qualitative trait means that in the presence of the chimeric gene of the invention, the phenotypic trait switches to a different discrete state when compared to a situation in which such gene is absent. A reduction of phenotypic expression of a nucleic acid may thus, a.o., be measured as a reduction in transcription of (part of) that nucleic acid, a reduction in translation of (part of) that nucleic acid or a reduction in the effect the presence of the transcribed RNA(s) or translated polypeptide (s) have on the eucaryotic cell or the organism, and will ultimately lead to altered phenotypic traits. It is clear that the reduction in phenotypic expression of a nucleic acid of interest, may be accompanied by or correlated to an increase in a phenotypic trait.

As used herein "a nucleic acid of interest" or a "target nucleic acid" refers to any particular RNA molecule or DNA sequence which may be present in a eucaryotic cell, particularly a plant cell.

As used herein "comprising" is to be interpreted as specifying the presence of the stated features, integers, steps or components as referred to, but does not preclude the presence or addition of one or more features, integers, steps or components, or groups thereof. Thus, e. g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structural defined, may comprise additional DNA regions etc.

It has unexpectedly been found by the inventors, that introduction of a chimeric gene capable of being transcribed into an RNA molecule with a nucleotide sequence comprising both the sense and antisense nucleotide sequence of a target gene, or part thereof, integrated in the nuclear genome of a plant cell, could efficiently and specifically reduce the phenotypic expression of that target gene. The reduction in phenotypic expression was more efficient and more predictable than observed when separate chimeric genes were introduced in similar cells with the target gene, encoding either sense or antisense RNA molecules.

In one embodiment of the invention, a method for reducing the phenotypic expression of a nucleic acid of interest, which is normally capable of being expressed in a eucaryotic cell, particularly a plant cell, is provided, comprising the steps of introducing a chimeric DNA comprising the following operably linked parts:
a) a promoter, operative in that cell, particularly a plant-expressible promoter;
b) a DNA region capable of being transcribed into an RNA molecule with a nucleotide sequence comprising
   i. a sense nucleotide sequence including at least 15 nt consecutive nucleotides having 100% sequence identity with at least part of a coding region of said nucleic acid of interest; and
   ii. an antisense nucleotide sequence including at least 15 consecutive nucleotides, having 100% sequence identity with the complement of the at least 15 consecutive nucleotides of the sense nucleotide sequence:
   wherein the RNA is capable of forming an artificial hairpin RNA structure with a double-stranded RNA stem by basepairing between the regions with sense and antisense nucleotide sequence such that at least said 15 consecutive nucleotides of the sense sequence basepair with said 15 consecutive nucleotides of the antisense sequence: wherein said nucleic acid of interest is a gene integrated in the genome of said eucaryotic cell, and wherein said method is not a method of treatment of the human or animal body by surgery or therapy, or a diagnostic method practised on the human or animal body; and optionally,
c) a DNA region involved in transcription termination and polyadenylation functioning in the suitable eucaryotic cells, particularly functioning in plant cells.

In a preferred embodiment of the invention, the RNA molecule transcribed from the chimeric gene, consists essentially of the hairpin RNA.

The order of the sense and antisense nucleotide sequence in the RNA molecule is thought not be critical.

Thus, in other words, the chimeric DNA has a transcribed DNA region, which when transcribed, yields an RNA molecule comprising an RNA region capable of forming an artificial stem-loop structure, wherein one of the annealing RNA sequences of the stem-loop structure comprises a sequence of at least 15 consecutive nucleotides having a sequence identity of at 100% with at least part of the nucleotide sequence of the nucleic acid of interest, and wherein the second of the annealing RNA sequences comprises a sequence of at least 15 consecutive nucleotides having a sequence identity of 100% with at least part of the complement of at least part of the nucleotide sequence of the nucleic acid of interest.

The RNA molecule may comprise several artificial hairpin structures, which may be designed to reduce the phenotypic expression of different nucleic acids of interest.

The nucleic acid of interest, whose phenotypic expression is targeted to be reduced, is a gene incorporated in the genome of a eucaryotic cell, particularly a plant cell. It will be appreciated that the means and methods of the invention can be used for the reduction of phenotypic expression of a gene which belongs to the genome of the cell as naturally occurring, (an endogenous gene), as well as for the reduction of phenotypic expression of a gene which does not belong to the genome of the cell as naturally occurring, but has been introduced in that cell (a transgene). The transgene can be introduced stably, and is integrated into the nuclear genome of the cell.

In another preferred embodiment, the nucleic acid of interest, whose phenotypic expression is targeted to be reduced is a viral nucleic acid, particularly a viral RNA molecule, capable of infecting a eucaryotic cell, particularly a plant cell. In this case, the phenotype to be reduced is the replication of the virus, and ultimately, the disease symptoms caused by the infecting virus.

The nucleotide sequence of the target nucleic acid corresponding to the sense nucleotide sequence is part of a DNA region which is transcribed, particularly a DNA region which is transcribed and translated (in other words a coding region). It is particularly preferred that the target sequence corresponds to one or more consecutive exons, more particularly is located within a single exon of a coding region.

The length of the sense nucleotide sequence may vary from 15 nucleotides (nt) up to a length equaling the length (in nucleotides) of the target nucleic acid. Preferably the total length of the sense nucleotide sequence is at least 15 nt, particularly at least about 50 nt, more particularly at least about 100 nt, especially at least about 150 nt, more especially at least about 200 nt, quite especially at least about 550 nt. It is expected that there is no upper limit to the total length of the sense nucleotide sequence, other than the total length of the target nucleic acid. However for practical reason (such as e. g. stability of the chimeric genes) it is expected that the length of the sense nucleotide sequence should not exceed 5000 nt, particularly should not exceed 2500 nt and could be limited to about 1000 nt.

The sense nucleotide sequence always includes a sequence of 15 consecutive nucleotides, particularly about 20 nt, more particularly about 50 nt, especially about 100 nt, quite especially about 150 nt with 100% sequence identity to the corresponding part of the target nucleic acid. Preferably, for calculating the sequence identity and designing the corresponding sense sequence, the number of gaps should be minimized, particularly for the shorter sense sequences.

The length of the antisense nucleotide sequence is largely determined by the length of the sense nucleotide sequence, and will preferably correspond to the length of the latter sequence. However, it is possible to use an antisense sequence which differs in length by about 10%. Similarly, the nucleotide sequence of the antisense region is largely determined by the nucleotide sequence of the sense region, and preferably is identical to the complement of the nucleotide sequence of the sense region. The antisense nucleotide sequences always includes a sequence of 15 consecutive nucleotides, particularly about 20 nt, more particularly about 50 nt, especially about 100 nt, quite especially about 150 nt with 100% sequence identity to the complement of a corresponding part of the sense nucleotide sequence. It is clear that the length of the stretch of the consecutive nucleotides with 100% sequence identity to the complement of the sense nucleotide sequence cannot be longer than the sense nucleotide sequence itself. Again, preferably the number of gaps should be minimized, particularly for the shorter antisense sequences.

The RNA molecule resulting from the transcription of the chimeric DNA may comprise a spacer nucleotide sequence located between the sense and antisense nucleotide sequence. In the absence of such a spacer sequence, the RNA molecule will still be able to form a double-stranded RNA, particularly if the sense and antisense nucleotide sequence are larger than 15 nucleotides and part of the sense and/or antisense nucleotide sequence will be used to form the loop allowing the base-pairing between the regions with sense and antisense nucleotide sequence and formation of a double stranded RNA. It is expected that there are no length limits or sequence requirements associated with the spacer region, as long as these parameters do not interfere with the capability of the RNA regions with the sense and antisense nucleotide sequence to form a double stranded RNA. In a preferred embodiment, the spacer region varies in length from 4 to about 200 bp, but as previously mentioned, it may be absent.

In a preferred embodiment, the hairpin RNA formed by the sense and antisense region and if appropriate the spacer region, is an artificial hairpin RNA. By "artificial hairpin RNA" or "artificial stem-loop RNA structure", is meant that such hairpin RNA is not naturally occurring in nature, because the sense and antisense regions as defined are not naturally occurring simultaneously in one RNA molecule, or the sense and antisense regions are separated by a spacer region which is heterologous with respect to the target gene, particularly, the nucleotide sequence of the spacer has a sequence identity of less than 75% with the nucleotide sequence of the target sequence, at the corresponding location 5' or 3' of the endpoints of the sense nucleotide sequence. A hairpin RNA can also be indicated as artificial, if it is not comprised within the RNA molecule it is normally associated with. It is conceivable to use in accordance with the invention a chimeric DNA whose transcription results in a hairpin RNA structure with a naturally occurring nucleotide sequence (which otherwise meets the limits as set forth in this specification) provided this hairpin RNA is devoid of the surrounding RNA sequences (not involved in the hairpin structure formation).

Although it is preferred that the RNA molecule comprising the hairpin RNA does not further comprise an intron sequence, it is clear that the chimeric DNA genes encoding such RNAs may comprise in their transcribed region one or more introns.

In fact, the inventors have unexpectedly found that inclusion of an intron sequence in the chimeric DNA genes encoding an RNA molecule comprising the hairpin RNA, preferably in the spacer region, and preferably in sense orientation, enhances the efficiency of reduction of expression of the target nucleic acid. The enhancement in efficiency may be expressed as an increase in the frequency of plants wherein silencing occurs or as an increase in the level of reduction of the phenotypic trait. In a particularly preferred embodiment, the intron is essentially identical in sequence to the *Flaveria trinervia* pyruvate orthophosphate dikinase 2 intron 2, more particularly, it comprises the sequence of SEQ ID No 7.

It has been observed that contrary to methods using either antisense or sense nucleotide sequences alone to reduce the phenotypic expression of a target nucleic acid (which generally depend on the dosage of sense or antisense molecule, and thus the chimeric genes encoding the sense and antisense molecules need to be under the control of relatively strong promoters) the method of the current invention does not rely on the presence of such strong promoter regions to drive the transcriptional production of the RNA comprising both the sense and antisense region. In other words, a whole range of promoters, particularly plant expressible promoters, is available to direct the transcription of the chimeric genes of the invention. These include, but are not limited to strong promoters such as CaMV35S promoters (e.g., Harpster et al., 1988). In the light of the existence of variant forms of the CaMV35S promoter, as known by the skilled artisan, the object of the invention can equally be achieved by employing these alternative CaMV35S promoters and variants. It is also clear that other plant-expressible promoters, particularly constitutive promoters, such as the opine synthase promoters of the Agrobacterium Ti- or Ri-plasmids, particularly a nopaline synthase promoter, or subterranean clover virus promoters can be used to obtain similar effets. Also contemplated by the invention are chimeric genes to reduce the phenotypic expression of a nucleic acid in a cell, which are under the control of single subunit bacteriophage RNA polymerase specific promoters, such as a T7 or a T3 specific promoter, provided that the host cells also comprise the corresponding RNA polymerase in an active form.

It is a further object of the invention, to provide methods for reducing the phenotypic expression of a nucleic acid in specific cells, particularly specific plant cells by placing the chimeric genes of the invention under control of tissue-specific or organ-specific promoters. Such tissue-specific or organ-specific promoters are well known in the art and include but are not limited to seed-specific promoters (e. g., W0WO89/03887), organ-primordia specific promoters (An et al., 1996), stem-specific promoters (Keller et al., 1988), leaf specific promoters (Hudspeth et al., 1989), mesophyl-specific promoters (such as the light-inducible Rubisco promoters), rootspecific promoters (Keller et al., 1989), tuber-specific promoters (Keil et al., 1989), vascular tissue specific promoters (Peleman et al., 1989), stamen-selective promoters (WO 89/10396, WO 92/13956), dehiscence zone specific promoters (WO 97/13865) and the like.

In another embodiment of the invention, the expression of a chimeric gene to reduce the phenotypic expression of a target nucleic acid can be controlled at will by the application of an appropriate chemical inducer, by operably linking the transcribed DNA region of the chimeric genes of the invention to a promoter whose expression is induced by a chemical compound, such as the promoter of the gene disclosed in European Patent publication ("EP") 0332104, or the promoter of the gene disclosed in WO 90/08826.

The chimeric gene (s) for reduction of the phenotypic expression of a target nucleic acid of interest in a cell, may be introduced either transiently, or may be stably integrated in the nuclear genome of the cell. In one embodiment, the chimeric gene is located on a viral vector, which is capable of replicating in the eucaryotic cell, particularly the plant cell (see e.g., WO 95/34668 and WO 93/03161).

The recombinant DNA comprising the chimeric gene to reduce the phenotypic expression of a nucleic acid of interest in a host cell, may be accompanied by a chimeric marker gene, particularly when the stable integration of the transgene in the genome of the host cell is envisioned. The chimeric marker gene can comprise a marker DNA that is operably linked at its 5' end to a promoter, functioning in the host cell of interest, particularly a plant-expressible promoter, preferably a constitutive promoter, such as the CaMV 35S promoter, or a light inducible promoter such as the promoter of the gene encoding the small subunit of Rubisco; and operably linked at its 3' end to suitable plant transcription 3' end formation and polyadenylation signals. It is expected that the choice of the marker DNA is not critical, and any suitable marker DNA can be used. For example, a marker DNA can encode a protein that provides a distinguishable color to the transformed plant cell, such as the A1 gene (Meyer et al., 1987), can provide herbicide resistance to the transformed plant cell, such as the bar gene, encoding resistance to phosphinothricin (EP 0,242,246), or can provide antibiotic resistance to the transformed cells, such as the aac(6') gene, encoding resistance to gentamycin (WO94/01560).

A recombinant DNA comprising a chimeric gene to reduce the phenotypic expression of a gene of interest, can be stably incorporated in the nuclear genome of a cell of a plant. Gene transfer can be carried out with a vector that is a disarmed Ti-plasmid, comprising a chimeric gene of the invention, and carried by Agrobacterium. This transformation can be carried out using the procedures described, for example, in EP 0 116 718.

Alternatively, any type of vector can be used to transform the plant cell, applying methods such as direct gene transfer (as described, for example, in EP 0 233 247), pollen-mediated transformation (as described, for example, in EP 0 270 356, WO85/01856 and US 4,684,611), plant RNA virus-mediated transformation (as described, for example, in EP 0 067 553 and US 4,407,956), liposome-mediated transformation (as described, for example, in US 4,536,475), and the like.

Other methods; such as microprojectile bombardment as described for com by Fromm, et al. (1990) and Gordon-Kamm et al. (1990), are suitable as well. Cells of monocotyledonous plants, such as the major cereals, can also be transformed using wounded and/or enzyme-degraded compact embryogenic tissue capable of forming compact embryogenic callus, or wounded and/or degraded immature embryos as described in W092/09696. The resulting transformed plant cell can then be used to regenerate a transformed plant in a conventional manner.

The obtained transformed plant can be used in a conventional breeding scheme to produce more transformed plants with the same characteristics or to introduce the chimeric gene for reduction of the phenotypic expression of a nucleic acid of interest of the invention in other varieties of the same or related plant species, or in hybrid plants. Seeds obtained from the transformed plants contain the chimeric genes of the invention as a stable genomic insert.

It is a further object of the invention to provide eucaryotic cells, preferably plant cells and organisms (preferably plants) comprising the chimeric genes for the reduction of the phenotypic expression of a target nucleic acid as described in the invention.

In its most straightforward embodiment, the RNA molecule comprising both the sense and antisense nucleotide sequences to at least part of a nucleic acid of interest, suitable for the methods of the invention, can be obtained by cloning two copies of a DNA region with the selected target sequence in inverted repeat orientation (preferably separated by a short DNA region which does not contain a transcription termination signal, and encodes the spacer sequence) under a suitable promoter. This chimeric DNA is then introduced in the host cell.

The methods and means of the invention can thus be used to reduce phenotypic expression of a nucleic acid in a eucaryotic cell or organism, particularly a plant cell or plant, for obtaining shatter resistance (WO 97/13865), for obtaining modified flower color patterns (EP 522 880, US 5,231,020), for obtaining nematode resistant plants (WO 92/21757, WO 93/10251, WO 94/17194), for delaying fruit ripening (WO 91/16440, WO 91/05865, WO 91/16426, WO 92/17596, WO 93/07275, WO 92/04456, US 5,545,366), for obtaining male sterility (WO 94/29465, WO89/10396 WO 92/18625), for reducing the presence of unwanted (secondary) metabolites in organisms, such as glucosinolates (WO97/16559) or chlorophyll content (EP 779 364) in plants, for modifying the profile of metabolites synthesized in a eucaryotic cell or organisms by metabolic engineering e.g. by reducing the expression of particular genes involved in carbohydrate metabolism (WO 92/11375, WO 92/11376, US 5, 365, 016, WO 95/07355) or lipid biosynthesis (WO 94/18337, US 5, 530, 192), for delaying senescence (WO 95/07993), for altering lignification in plants (WO 93/05159, WO 93/05160), for altering the fibre quality in cotton (US 5, 597,718), for increasing bruising resistance in potatoes by reducing polyphenoloxidase (WO 94/03607), etc.

The methods of the invention will lead to better results and/or higher efficiencies when compared to the methods using conventional sense or antisense nucleotide sequences and it is believed that other sequence-specific mechanisms regulating the phenotypic expression of target nucleic acids might be involved and/or triggered by the presence of the double-stranded RNA molecules described in this specification.

A particular application for reduction of the phenotypic expression of a transgene in a plant cell, inter alia, by antisense or sense methods, has been described for the restoration of male fertility, the latter being obtained by introduction of a transgene comprising a male sterility DNA (WO 94/09143, WO 91/02069). The nucleic acid of interest is specifically the male sterility DNA. Again, the processes and products described in this invention can be applied to these methods in order to arrive at a more efficient restoration of male fertility.

The methods and means of the invention; involving RNA molecules comprising a hairpin RNA encoded by the chimeric genes, have proven to be particularly suited for the modification of the composition of oil content in plants, particularly in seeds. Particularly preferred plants are crop plants used for oil production such as but not limited to oilseed rape (Brassica juncea, napus, rapa, oleracea, campestris), corn, cotton, groundnut, sunflower, castor beans, flax, coconut, linseed, soybean. Preferred target genes are the desaturase genes, particularly A12 desaturase encoding genes such as those encoded by the Fad2 genes, especially the genes whose nucleotide sequence can be found in the Genbank Database under accession number AF123460 (from Brassica carinata), AF 12042841 (Brassica rapa), L26296 (Arabidopsis thaliana) or A65102 (Corylus avellana). It is clear that it is well within the reach of the person skilled in the art to obtain genes homologous to the disclosed fad2 genes from other species e. g. by hybridization and/or PCR techniques.

Preferred embodiments for the configuration of sense and antisense nucleotide sequences, particularly concerning length and sequence are as mentioned elsewhere in this specification. Also, preferred embodiments for chimeric genes encoding hairpin containing RNA molecules, particularly concerning promoter elements are as described elsewhere in the specification. For this application, it is particularly preferred that the promoters are seed-specific promoters.

The artificial hairpin RNA comprising RNA molecule thus can comprises part of a Δ12 desaturase encoding ORF in sense orientation and a similar part in antisense orientation, preferably separated by a spacer sequence. The chimeric gene encoding the artificial hairpin RNA may comprises the nucleotide sequence of SEQ ID No 6 or a similarly constructed nucleotide sequence based on the aforementioned Brassica fad2 genes.

Preferably the chimeric gene encoding the artificial hairpin RNA is transcribed under control of a seed-specific promoter, particularly under control of the FPI promoter as described elsewhere in this application.

A reduction of the expression of Δ12 desaturase gene in oil containing plants leads to increase in oleic acid and a concomitant decrease in linolenic acid and linoleic acid. A higher frequency of plants with oil wherein the increase in oleic acid and concomitant decrease in linolenic and linoleic acid is significant is found using the means and methods of the invention than in transgenic plants harboring common cosuppression constructs. Moreover the absolute levels of increase, respectively decrease are higher respectively lower than in transgenic plants harboring common cosuppression constructs.

Using the means and methods of the invention, it is thus possible to obtain plants and seeds, comprising an oil of which the composition after crushing and extracting has an increased oleic acid content (expressed as a percentage of the total fatty acid composition), particularly a three fold increase, when compared with control plants.

It is expected that using the methods and means of the invention, transgenic Brassica plant can be obtained, whose seeds comprise an oil wherein the oleic acid content exceeds 90% of the total fatty acid contents.

The methods and means of the invention will be suited for obtaining virus resistance, in eucaryotic cells or organisms.

The methods and means of the invention further allow the use of viral genes, hitherto unused for obtaining virus resistant plants in addition to the commonly used coat protein genes or replicase genes.

It is clear that the invention will be especially suited for the reduction of phenotypic expression of genes belonging to multigene families.

It is also clear that the methods and means of the invention are suited for the reduction of the phenotypic expression of a nucleic acid in all plant cells of all plants, whether they are monocotyledonous or dicotyledonous plants, particularly crop plants such as but not limited to corn, rice, wheat, barley, sugarcane, cotton, oilseed rape, soybean, vegetables (including chicory, brassica vegetables, lettuce, tomato), tobacco, potato, and sugarbeet, but also plants used in horticulture, floriculture or forestry. The means and methods of the invention will be particularly suited for plants which have complex genomes, such as polyploid plants.

It is expected that the chimeric RNA molecules produced by transcription of the chimeric genes described herein, can spread systemically throughout a plant, and thus it is possible to reduce the phenotypic expression of a nucleic acid in cells of a non-transgenic scion of a plant grafted onto a transgenic stock comprising the chimeric genes of the invention (or vice versa) a method which may be important in horticulture, viticulture or in fruit production.
The following non-limiting Examples describe the construction of chimeric genes for the reduction of the phenotypic expression of a nucleic acid of interest in a eucaryotic cell and the use of such genes. Unless stated otherwise in the Examples, all recombinant DNA techniques are carried out according to standard protocols as described in Sambrook et al. (1989) Molecular Cloning : A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK.

Throughout the description and Examples, reference is made to the following sequences:

| | |
|---|---|
| SEQ ID N° 1: | sequence of the Potato virus Y fragment of the Nia gene used for the construction of various sense and antisense constructs, for obtaining virus resistance. |
| SEQ ID N° 2: | sequence of the coding region of the Gusd CoP construct of Example 1. |
| SEQ ID N° 3: | sequence of a modified 5' untranslated region (5'UTR) from Johnsongrass mosaic virus |
| SEQ ID N° 4: | sequence of the Subterranean clover virus promoter No 4 with S7 enhancer. |
| SEQ ID N° 5: | sequence of the Subterranean clover virus double enhancer promoter No 4. |
| SEQ ID N° 6: | sequence of the CoP construct for the Δ12 desaturase gene expression inhibition. |
| SEQ ID N° 7: | sequence of the Flaveria trinervia pyruvate orthophosphate dikinase intron |

**The following free text is included in the sequence listing :**
<223> fragment of the NIa ORF
<223> Description of Artificial Sequence:coding region of the Gusd CoP construct
<223> deficient Gus coding region
<223> antisense to the 5' end of the Gus coding region
<223> Description of Artificial Sequence:5'UTR of Johnson mosaic virus
<223> Description of Artificial Sequence:Subterannean clover virus S4 promoter with S7 enhancer
<223> Description of Artificial Sequence: subterranean clover virus promoter S4 with S4 enhancer
<223> Description of Artificial Sequence: coding sequence of the desaturase CoP construct
<223> corresponding to the 5' end of the delta12-desaturase (fad2) coding region, in sense orientation
<223> corresponding to the 5' end of the delta12-desaturase (fad2) coding region, in anti sense orientation
<223> Description of Artificial Sequence: intron 2 of the Flaveria trinervia puryvate orthophosphate dikinase

The following examples are presented in order to more fully illustrate the preferred embodiments of the invention. They should in no way be construed, however, as limiting the broad scope of the invention.

### EXAMPLE 1

### Experimental procedures

### Gene construction

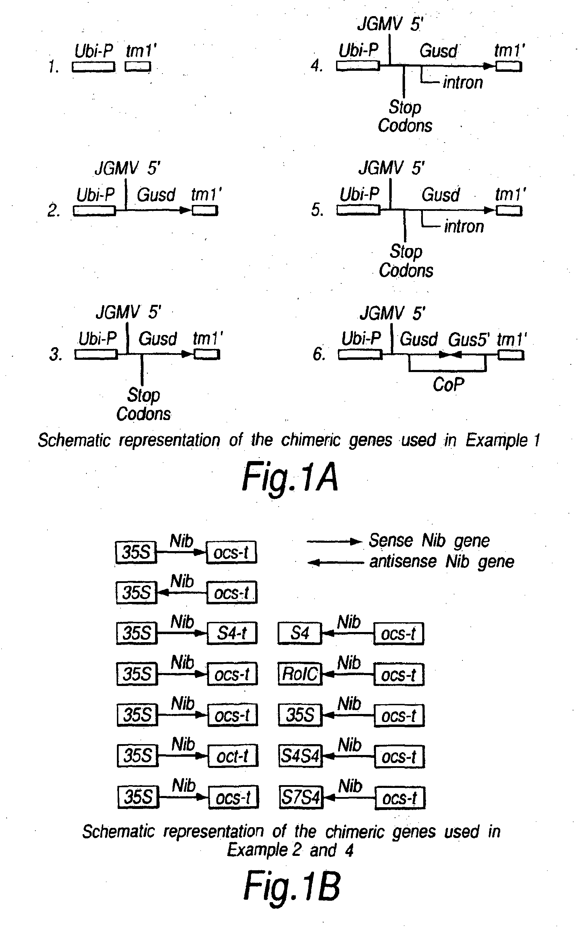

Standard gene cloning methods (Sambrook et al. 1989) were used to make the chimeric genes. A schematic representation of the constructs used is shown in Figures 1A and 1B.

The components for these constructs were:
* Cauliflower mosaic virus 35S promoter from the Cabb-JI isolate(35S) (Harpster *et al.,* 1988)
* Octopine synthase terminator (ocs-t) (MacDonald *et al.,* 1991)
* Subterranean clover virus promoter No 4 (S4) (WO 9606932)
* Subterranean clover virus terminator No 4 (s4t) (WO 9606932)
* Subterranean clover virus double enhancer promoter No 4 (S4S4) (SEQ ID N° 5)
* Subterranean clover virus promoter No 4 with S7 enhancer (S7S4) (SEQ ID N° 4)
* Maize ubiquitin promoter (Ubi) (Christensen and Quail, 1996)
* Agrobacterium tumour morphology 1 gene terminator (tm1') (Zheng *et al.,* 1991)
* the Nia gene of an Australian strain of Potato virus Y (Nia) (SEQ ID N° 1)
* a dysfunctional β-glucuronidase open reading frame encoding DNA (Gusd)(SEQ ID N° 2 from nucleotide 1 to nucleotide 1581)
* a modified 5' untranslated region (5'UTR) from Johnsongrass mosaic virus (JGMV5') (SEQ ID N° 3).
   This contains insertion of a *Nco*I site at the ATG start codon followed by three stop codons in frame, and a *Pst*I site (for insertion of the intron as in constructs 4 and 5 of Figure 1A). In vector constructs 2 and 6 of Figure 1A, the Gusd open reading frame is inserted in at the *Nco*I site, removing the stop codons; in all other constructs of Fig 1A it is inserted downstream of the *Pst*I site.
* a castor bean catalase intron (Ohta et al., 1990) as modified by Wang *et al*.(1997) ("intron").

The chimeric genes were constructed by operably assembling the different parts as schematically indicated in Figure 1A or Figure 1B and inserting the obtained chimeric genes in the T-DNA vectors pART27 and pART7 vectors (Gleave, 1992) between the left T-DNA border and the chimeric plant expressible *neo* gene.

The DNA encoding a dysfunctional β-glucuronidase open reading frame (GUSd) was obtained by deleting from a *gus* coding region the sequence between the two EcoRV restriction sites. For the construction of the chimeric gene encoding the RNA molecule comprising both sense and antisense nucleotide sequence to a β-glucuronidase gene, a sequence was added to the Gusd gene to be allow base pairing the 5'end over 558 bases resulting in a sequence as represented in SEQ ID N° 2. This sequence was cloned between the maize ubiquitin promoter and the tm1' terminator and inserted in a T-DNA vector.

T-DNA vectors were constructed which comprised a first and a second chimeric virus resistance gene, wherein the first chimeric gene consisted of:
1. a CaMV 35S promoter sequence, coupled to
2. in sense orientation, the nucleotide sequence from PVY encoding either
   - Vpg protein (see *e.g*., Genbank Accession Nr ZZ9526 from nucleotide 1013 to nucleotide 1583), or
   - part of the Cl protein (see *e*.*g*., Genbank Accession Nr M95491 from nucleotide 3688 to nucleotide 4215) or
   - Protease (Pro) (see *e.g.*, EMBL Accession Nr D00441 from nucleotide 5714 to nucleotide 7009), followed by
3. the S4 terminator from subterranean clover mosaic virus, as described above.
The second chimeric gene consists of
1. a S4 promoter as described above, coupled to
2. in anti-sense orientation, the nucleotide sequence from PVY encoding either
   - Vpg protein , or
   - Cl protein or
   - Protease, followed by
3. the octopine synthase terminator as described above.
The sense and antisense sequences within one T-DNA vector were derived from the same PVY coding region.

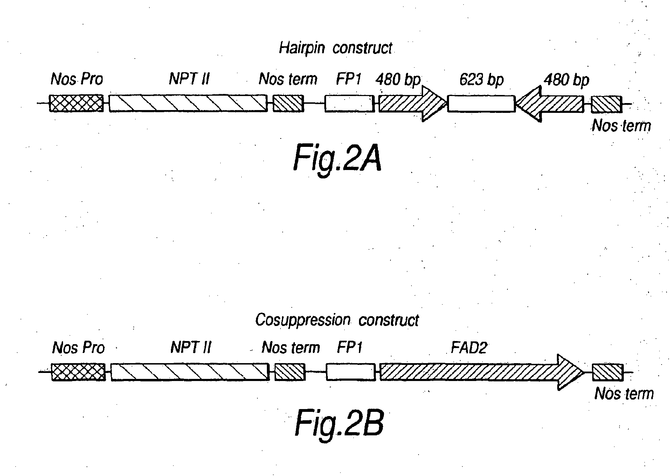

Also, T-DNA vectors were constructed for use in altering the fatty acid composition in oil (see Fig 2), comprising
1. a FP1 promoter (truncated seed specific napin promoter, containing sequences between -309 and +1, as described in Stalberg et al; linked to
2. the nucleotide sequence of SEQ ID No 6, comprising the 480 bp located 5' in the ORF encoding the Δ12 desaturase from *Arabidopsis thaliana* (Fad2) in sense orientation and in antisense orientation, linked by a 623 bp spacer sequence; followed by
3. the terminator from the nopaline synthase gene.

In addition, T-DNA vectors were constructed to evaluate the influence of a presence of an intron sequence in the chimeric genes encoding CoP constructs. To this end, constructs were made comprising:
1. a CamV35S promoter, followed by
2. the protease encoding ORF from PVY (see above) in sense orientation;
3. the sequence of SEQ ID No 7 (encoding the *Flaveria trinervia* pyruvate orthophospate dikinase intron 2)
4. the protease encoding ORF from PVY in antisense orientation; and
5. the octopine synthase gene terminator.

### Plant transformation

*Nicotiana tabacum* (W38) tissue was transformed and regenerated into whole plants essentially as described by Landsman *et al.* (1988). Rice *(Oryza sativa)* was transformed essentially as described by Wang *et al.* (1997).

### Rice supertransformation

Mature embryos from a rice plant expressing GUS and hygromycin phosphotransferase (HPT) activity were excised from mature seed and placed on callus inducing media for 7 weeks. Calli were recovered from these cultures, incubated with Agrobacteria containing various binary vector constructs for 2 days, then placed on callusing media containing hygromycin, bialaphos and Timentin™. During the next four weeks hygromycin and bialaphos resistant calli developed. These callus lines were maintained on hygromycin and bialaphos containing media for a further 2 months before being assayed for GUS activity.

### GUS Assay

Rice calli were tested for GUS activity using the histochemical stain X-glucuronide or the fluorogenic substrate 4-methyl-umbeliferone glucuronide (MUG) essentially as described by Jefferson *et al.* (1987).

### EXAMPLE 1. Comparison of chimeric genes comprising only antisense, only sense, or both sense and antisense (Complimentary Pair (CoP)) sequence for reduction in phenotypic expression of an integrated β-glucuronidase gene.

Transgenic rice tissue expressing β-glucuronidase (GUS) from a single transgene (and hygromycin resistance from a *hph* gene) (lines V10-28 and V10-67) was supertransformed using vectors that contained the *bar* gene conferring phosphinothricin resistance and various sense, antisense and CoP constructs (see Figure 1A) derived from a crippled GUS (GUSd) gene. The supertransformed tissue was maintained on hygromycin and bialaphos selection media for 3 weeks then analyzed for GUS activity. A crippled GUS gene was used so that expression from this gene would not be superimposed on the endogenous GUS activity.

The figures in Table 2 represent the rate of MU production measured by absorption at 455 nm, with excitation at 365 nm of 1.5 µg of total protein in a reaction volume of 200 µl. The rate was measured over 30 min at 37°C. The reading for non-transgenic rice calli was 0.162. The figures in bracket which follow the description of the introduced construct refer to Figure 1A.

The results (Table 2) showed that supertransformation with the binary vector containing the bar gene without the GUSd gene had no silencing effect on the endogenous GUS activity. Supertransformation with GUSd in a sense or antisense orientation, with or without an intron or an early stop codon, showed some degree of reduction (in about 25% of the analyzed calli) of the endogenous GUS activity (see last two rows in Table 2 representing the percentage of analyzed calli with a MUG assay reading of less than 2.000). However, supertransformation with a CoP construct gave in about 75% to 100% of the analyzed calli, reduction of the endogenous GUS activity. This CoP construct was designed so that the 3' end of the mRNA produced could form a duplex with the 5' end of the transcript to give a "pan-handle" structure.

These data show that a complimentary pair can be made using one self-annealing transcript, that this design is much more effective than a conventional sense or antisense construct, and that the approach can be used to reduce the phenotypic expression of genes present in a plant cell.

**Table 2. MUG assay of Supertransformed Rice Calli**

| | Vector cassette (1) | Sense (2) | Sense + Stop (3) | Sense + stop+ intron (4) | Antisense + stop + intron (5) | Inverted repeat CoP (6) |
|---|---|---|---|---|---|---|
| V10-28 | 121,0 | 97,45 | 38,43 | 38,88 | 0,290 | 0,565 |
| | 45,58 | 6,637 | 64,16 | 115,5 | 0,572 | 0,316 |
| | 99,28 | 71,60 | 149,2 | 133,0 | 37,2 | 0,351 |
| | 26,17 | 0,224 | 0,955 | 98,46 | 53,94 | 0,210 |
| | 92,21 | 0,321 | 68,32 | 0,502 | 105,5 | 0,701 |
| | 108,8 | 5,290 | 105,6 | 39,35 | 56,73 | 0,733 |
| | 6,432 | 0,9460 | 136,6 | 1,545 | 60,36 | 2,103 |
| | 90,80 | 32,44 | 140,4 | 10,36 | 71,12 | 119,8 |
| | 98,24 | 128,8 | 62,38 | 111,6 | 13,17 | 0,717 |
| | 93,76 | 31,28 | 17,79 | 14,42 | 0,424 | 0,398 |
| | | 5,023 | | 88,06 | 26,98 | 0,315 |
| | | 40,27 | | 52,28 | 115,5 | 0,270 |
| | | 36,40 | | 30,26 | 149,7 | 16,78 |
| | | 53,24 | | 107,5 | 66,75 | 67,28 |
| | | 29,97 | | 26,75 | 145,8 | 0,217 |
| | | 89,06 | | 105,1 | 0,534 | 0,208 |
| | | 0,256 | | 135,1 | 9,4 | |
| | | 68,23 | | 95,04 | 35,33 | |
| | | 5,481 | | 71,5 | | |
| | | | | | | |
| V10-67 | 318,8 | 93,43 | 0,199 | 31,82 | 1,395 | 0,472 |
| | 109,5 | 73,19 | 0,197 | 58,08 | 152,4 | 0,256 |
| | 30,35 | 128,1 | 0,157 | 56,32 | 67,42 | 0,296 |
| | 40,04 | 1,506 | 128 | 44,62 | 12,11 | 0,452 |
| | 228 | 140,6 | 130,3 | 0,454 | 0,668 | 0,422 |
| | 23,05 | 1,275 | 196,2 | 17,32 | 23,34 | 0,196 |
| | 241,2 | 0,272 | 12,43 | 73,2 | 76,10 | 0,294 |
| | 118,5 | 0,209 | 140,0 | 20,32 | 130,1 | 0,172 |
| | 11,27 | 42,05 | 90,13 | 107,4 | 0,841 | 0,436 |
| | 110,6 | 117,5 | 157,4 | 0,453 | 66,12 | 0,398 |
| | 19,29 | 118,9 | 0,518 | 87,81 | 136,9 | 0,242 |
| | 121,0 | 21,44 | 0,231 | 0,299 | 67,92 | |
| | 115,1 | 155,0 | 116,1 | 0,206 | 50,32 | |
| | 77,1 | 190,9 | 43,18 | 12,47 | 170,3 | |
| | 106,1 | 0,773 | 31,06 | 0,213 | 108,9 | |
| | 73,12 | 0,146 | | 11,15 | 1,241 | |
| | 29,97 | | | 19,22 | 4,092 | |
| | 50,11 | | | | 169,6 | |
| | 80,34 | | | | 76,88 | |
| | 117,8 | | | | 22,08 | |
| | 159,1 | | | | 91,6 | |
| | 67,52 | | | | 7,855 | |
| | 92,32 | | | | 69,76 | |
| | 27,97 | | | | 0,822 | |
| V10-28 | 0% | 21% | 10% | 10,5% | 22% | 75% |
| V10-67 | 0% | 37,5% | 33% | 29,5% | 21% | 100% |

### EXAMPLE 2. (Comparative) Comparison of the efficiency of using chimeric genes comprising only antisense genes, only sense genes, or both genes simultaneously for obtaining virus resistance in transgenic plants.

Gene constructs were made using the PVY protease encoding sequence (SEQ ID N 1) in a sense orientation, an antisense orientation and in a complimentary pair (CoP) orientation, where the T-DNA comprised both the sense and antisense chimeric genes each under control of their own promoter. In all three arrangements the CaMV35S promoter was used. Five different versions of CoP constructs were made in which the second promoter was either the CaMV35S promoter, the S4 promoter, the double S4 promoter, the S7 enhanced S4 promoter, or the vascular specific *roIC* promoter (see Figure 1 B).

These constructs were transformed into tobacco (via *Agrobacterium* mediated DNA transfer) and approximately 25 independently transformed plants were recovered per chimeric gene construct. The transgenic plants were transferred to soil and maintained in the greenhouse. About 1 month after transplanting to soil, the plants were inoculated manually with potato virus Y, using standard application methods. Two and four weeks later the plants were scored for virus symptoms. The results (Table 3) showed that after 1 month, 2 on 27 plants comprising only the sense gene, and 1 on 25 plants comprising only the antisense gene showed no symptoms.

In contrast respectively 11 on 24 (35S-Nia/S4-antisenseNia construct), 7 on 25 (35S-Nia/RoIC-antisenseNia construct), 10 on 27 (35S-Nia/35S-antisenseNia), 7 on 26 (35S-Nia/S4S4-antisenseNia construct), and 7 on 25 (35S-Nia/S7S4antisenseNia construct) plants which contained both the sense and antisense genes, showed no symptoms. Plants that showed no symptoms were considered to be showing extreme resistance to PVY. They continued to show no symptoms for a further 2 months of monitoring (indicated as Extreme Resistant (ER) in Table 3).
Some other plants, particularly those containing CoP constructs, showed a delay and restriction of symptoms. They showed no symptoms 2 weeks after inoculation but showed some minor lesions in some plants after 4 weeks. These plants were clearly much less effected by PVY than non-transgenic or susceptible tobaccos and were scored as resistant (indicated as ER* in Table 3).

**Table 3 Resistance to PVY infection of transgenic tobacco plants comprising either the sense chimeric PVY protease construct, the antisense chimeric PVY protease construct, or both (different CoP constructs).**

| Sense gene | Antisense gene | Extreme Resistant plants (ER) | "Resistant" plants (ER*) | Total number of transgenic plants |
|---|---|---|---|---|
| 35S-Nia | | 2 | 2 | 27 |
| | 35S-AntisenseNia | 1 | 0 | 25 |
| 35S-Nia | 35S- AntisenseNia | 10 | 2 | 27 |
| 35S-Nia | S4-AntisenseNia | 11 | 2 | 24 |
| 35S-Nia | RoIC-AntisenseNia | 7 | 3 | 25 |
| 35S-Nia | S4S4-AntisenseNia | 7 | 7 | 26 |
| 35S-Nia | S7S4-AntisenseNia | 7 | 4 | 25 |

The data show that using CoP constructs results in a much higher frequency of transgenic plants with extreme resistance and resistance than by using either sense or antisense constructs alone.

Next, the copy number of the transgenes in the virus resistant transgenic plants was determined. Therefore, DNA was extracted from all the transgenic plants showing extreme resistance or resistance. DNA was also extracted from five susceptible plants for each construct. The DNA was examined for gene copy number using Southern analysis. The data (Table 4) showed that the genomes of some of the CoP plants showing extreme resistance, particularly the 35S-Nia/S4-AntisenseNia plants, only contained a single copy of the gene construct.

**Table 4. Copy number of transgenes comprising sense chimeric PVY protease construct, the antisense chimeric PVY protease construct, or both (different CoP constructs) in extreme resistant, resistant and susceptible plants.**

| Sense gene | Antisense gene | Extreme Resistant plants (ER) | "Resistant" plants (ER*) | Susceptible plants |
|---|---|---|---|---|
| 35S-Nia | | 6 | 1 | 1/1/1/1/1 |
| | 35S-AntisenseNia | 4 | - | 1/8/0/2/1 |
| 35S-Nia | 35S-AntisenseNia | 3/1/2/3/6/3/2/4/2/3 | 1/1 | 1/2/2/6/1 |
| 35S-Nia | S4-AntisenseNia | 2/4/1/3/2/4/6/1/1/3/1 | 2/6 | 5/8/2/3 |
| 35S-Nia | RoIC-AntisenseNia | 6/7/6/7/7/7/6 | 2/1 | 2/2/2/1/2 |
| 35S-Nia | S4S4-AntisanseNia | 1/214/5/2/2/2 | 1/1/2/1/1/1 | 1/1/7/1/1 |
| 35S-Nia | S7S4-AntisenseNia | 2/4/12/5/2/2/7 | 3/2/1/2 | 1/1/3/1/1 |

### EXAMPLE 3. (Comparative) Inheritance of extreme resistance in plants from Example 2.

Plants from Examples 2 were allowed to self-fertilize and their seeds were collected. Seeds originating from plants showing extreme resistance and low transgene copy number for CoP constructs 35S-Nia/S4-AntisenseNia and 35SNia/35S-AntisenseNia, and seeds from the sense and the antisense plants showing extreme resistance, were germinated and grown in the glasshouse. Plants were also grown from seed collected from two susceptible CoP lines, two susceptible sense gene only lines and two susceptible antisense gene onty lines. Twenty plants from each line were selected for overall uniformity of size and development stage, put into individual pots, allowed to recover for one week, then inoculated with PVY. The plants were scored for virus symptoms 2,4, and 7 weeks after inoculation. The results (Table 5) showed that all eight plant lines of 35S-Nia/S4-antisenseNia and 35S-Nia/35S-antisenseNia containing one or two gene copies showed an about 3: 1 segregation ratio of extreme resistance: susceptible. The progeny of the single antisense gene only line that had given extreme resistance at To, and the progeny of the extremely resistant sense plant containing one gene copy, gave abnormal segregation ratios (2: 18; ER: susceptible). The progeny of the one sense plant that gave extreme resistance and contained 6 gene copies gave a ∼3:1 ratio (ER: susceptible). All the progeny of the susceptible Tₒ plants showed complete susceptibility to PVY.

These data show extreme resistance from CoP constructs gives stable expression of the resistance which is inherited in a Mendelian way. This also indicates that, in these lines the PVY CoP gene loci are-100% effective at conferring extreme resistance whereas the transgene loci in the antisense line and one of the two sense lines are only partially effective at conferring extreme resistance.

**Table 5.**

| | | 35S Sense Nia and S4 Antisense Nia | | 35S Sense Nia and S4 Antisense Nia | | 35S Sense Nia | | 35SAntisense Nia | |
|---|---|---|---|---|---|---|---|---|---|
| | | Copy N° | T1 ER:S | Copy N° | T1 ER:S | Copy N° | T1 ER:S | Copy N° | T1 ER:S |
| ER plant | 1 | 1 | 15:5 | 1 | 16:4 | 6 | 17:3 | 4 | 2:18 |
| | 2 | 1 | 12:8 | 2 | 15:5 | 1 | 2:18 | | |
| | 3 | 1 | 14:6 | 2 | 16:4 | | | | |
| | 4 | 1 | 15:5 | 2 | 16:4 | | | | |
| Susceptible Plant | | | | | | | | | |
| | 1 | 8 | 0:20 | 1 | 0:20 | 1 | 0:20 | 1 | 0:20 |
| | 2 | 2 | 0:20 | 1 | 0:20 | 1 | 0:20 | 8 | 0:20 |

### EXAMPLE 4: (Comparative) Extreme virus resistant transgenic tobacco with different components (sense gene and antisense gene) in different loci within the transgenic plant.

PVY susceptible plants containing the sense transgene (which contained single transgene copies; see Table 6) were crossed with PVY susceptible plants containing the antisense transgene (which had also been analyzed for copy number by Southern analysis; see Table 6). Twenty of the resulting progeny per cross were propagated in the glasshouse, then inoculated with PVY and scored for virus infection as described in Example 2. The progeny from the crosses (between single genes/loci containing plants) would be expected to be in the following ratio: 1/4 sense gene alone, ¼ antisense gene alone, ¼ comprising both sense and antisense genes, and ¼ comprising no genes at all. The results (Table 4) show that, with one exception, a proportion of the progeny from all the successful crosses showed extreme resistance, whereas none of the progeny from selfed sense or selfed antisense plants showed extreme resistance. The one cross that gave no extremely resistant progeny was derived from the parent plant Antisense 2 (As2) which contained 8 copies of the antisense gene. All twenty progeny plants from crosses Sense 1 (S1)(male) × Antisense 1 (As1) (female) and Sense 3 (S3) (female) × Antisense 4 (As4) (male) were examined by Southern analysis. The results showed that in both crosses, the plants that showed extreme resistance (or in one case resistance) contained both the sense and antisense genes, whereas plants with no transgenes (nulls), or sense or antisense genes alone, were all susceptible to PVY. To further confirm this absolute correlation between the presence of a complimentary pair (sense with antisense genes) within a plant and extreme resistance, all progeny plants showing extreme resistance were analyzed by Southern blots. The results showed that every extremely resistant or resistant plant contained both sense and antisense genes.

These data show that a complimentary pair gives resistance or extreme resistance even when the genes encoding the sense and antisense genes are not co-located in the genome. The "complimentary pair phenomenon" is not simply due to increased transgene dosage as it would be expected that ¼ of the selfed progeny would be homozygous and thus have double the gene dosage, yet they were susceptible.

**Table 6: PVY resistance of the progeny plants resulting from crosses between susceptible transgenic tobacco plants comprising the 35S-senseNia gene (S-lines) and susceptible transgenic tobacco plants comprising the 35S-antisenseNia gene (As-lines).**

| Male parent | Female parent | Extreme Resistant plants (ER) | "Resistant" plants (ER*) |
|---|---|---|---|
| S1 | As1 | 8 | |
| S1 | As4 | 6 | 5 |
| S2 | As4 | 1 | 3 |
| S3 | As4 | 3 | 3 |
| S4 | As1 | 7 | 1 |
| S3 | As5 | 1 | 2 |
| S4 | As2 | 0 | |
| S4 | As4 | 2 | 0 |
| S5 | As4 | 9 | 4 |
| S5 | As5 | 2 | 3 |
| As4 | As4 | 0 | |
| As5 | As5 | 0 | |
| S2 | S2 | 0 | |
| S4 | S4 | 0 | |

Extreme resistant plants showed no symptoms of PVY infection after 7 weeks. Resistant plants showed very minor lesions 7 weeks after PVY infection. S1, S2, S3, S4 and S5 are PVY susceptible transgenic tobacco plants comprising the 35S-senseNia gene construct which all have one copy of the transgene integrated.

As1, As2, As4 and As5 are PVY susceptible transgenic tobacco plants comprising the 35S-antisenseNia gene construct which have respectively 1, 8, 2 and 1 copies of the transgene integrated.

### EXAMPLE 5. (Comparative) Evaluation of the use of different viral genes as target nucleic acid sequences in obtaining extreme virus resistant genes.

The T-DNA vectors comprising first and second chimeric virus resistance genes based on sequences derived from the coding region for protease, Vpg or Cl proteins from PVY as described in this application, were used to obtain transformed tobacco plants, which were subsequently challenge with PVY. The results are summarized in the following table:

**Table 7.**

| Construct | Number of immune plants/ Number of independent transgenic plants | |
|---|---|---|
| | Replica 1 | Replica 2 |
| 35S-Pro sense /S4-Pro antisense | 11/24 | 7/25 |
| 35S-Vpg sense /S4-Vpg antisense | 8/20 | 6/18 |
| 35S-Cl sense /S4-Cl antisense | 2/23 | 1/20 |

### Example 6. Intron enhanced silencing

The T-DNA vectors comprising the chimeric genes encoding the CoP constructs wherein an intron (*Flaveria trinervia* pyruvate orthophosphate dikinase intron 2) has been inserted in either the sense orientation or the antisense orientation, between the sense and antisense sequences corresponding to the protease encoding ORF from PVY (as described elsewhere in this application) were used to obtain transformed tobacco plants, which were subsequently challenge with PVY. The results are summarized in the following table:

**Table 8.**

| Construct | Number of immune plants/ Number of independent transgenic plants |
|---|---|
| 35S-Pro(sense)-intron(sense)-Pro(antisense)-Ocs-t | 22/24 |
| 35SPro(senseb)-intron(antisense)-Pro(antisense)-Ocs-t | 21/24 |

### EXAMPLE 7. Modifying oil profile using CoP constructs in Arabidopsis.

T-DNA vectors for modifying the fatty acid composition in oil, extracted from crushed seeds as described elsewhere in this application were used to introduce the chimeric gene encoding the CoP construct for reducing the expression (see Fig 2A; SEQ ID No 6) the Δ12 desaturase gene (Fad2) in *Arabidopsis thaliana.*

For comparison of the efficiency, transgenic *Arabidopsis* plants were generated wherein the Fad2 gene expression was reduced by a plain cosuppression construct, comprising the FPI seed-specific promoter coupled to the complete ORF from the Δ12 desaturase gene (Fad2) in *Arabidopsis thaliana* and the nopaline synthase promoter (see Fig 2B).

As control plants, transgenic *Arabidopsis* transformed by unrelated T-DNA constructs were used.

Seeds were harvested, crushed and extracted and the percentage of the major fatty acids in the oil was determined by methods available in the art. The results, which are the mean of two readings, are summarized in Table 9.

**Table 9.**

| **Sample** | **Peak Names ------->** | | | | | | | | | | | | |
|---|---|---|---|---|---|---|---|---|---|---|---|---|---|
| **Name** | **Myristic** | **Palmitic** | **Palmitoleic** | **Stearic** | **Oleic** | **Linoleic** | **Linolenic** | **20:0** | **20:1** | **22:0** | **22:1** | **24:0** | **C18:1/(C18:2 + C18:3)** |
| Hairpin 1.1 | 0.00 | 6.06 | 0.52 | 3.21 | 56.65 | 7.50 | 6.82 | 1.46 | 16.02 | 0.00 | 1.76 | 0.00 | 3.95 |
| Hairpin 1.2 | 0.12 | 6.86 | 0.39 | 3.40 | 51.28 | 10.00 | 8.73 | 1.64 | 15.60 | 0.00 | 1.97 | 0.00 | 2.74 |
| Hairpin 1.3 | 0.11 | 8.47 | 0.50 | 3.49 | 21.64 | 28.99 | 18.51 | 2.02 | 14.19 | 0.00 | 2.09 | 0.00 | 0.46 |
| Hairpin 1.4 | 0.00 | 6.14 | 0.50 | 3.37 | 51.70 | 9.77 | 8.02 | 1.73 | 16.04 | 0.00 | 2.05 | 0.67 | 2.91 |
| Hairpin 2.1 | 0.06 | 5.19 | 0.43 | 3.33 | 54.84 | 5.52 | 7.76 | 1.77 | 18.50 | 0.34 | 1.83 | 0.45 | 4.13 |
| Hairpin 2.2 | 0.04 | 7.67 | 0.46 | 3.75 | 19.60 | 28.29 | 18.64 | 2.55 | 15.96 | 0.19 | 2.28 | 0.56 | 0.42 |
| Hairpin 3.1 | 0.00 | 7.99 | 0.53 | 3.62 | 19.52 | 28.41 | 19.24 | 2.32 | 15.14 | 0.00 | 2.23 | 0.99 | 0.41 |
| Hairpin 3.2 | 0.09 | 7.00 | 0.54 | 3.69 | 49.02 | 11.03 | 9.64 | 1.71 | 14.94 | 0.00 | 1.72 | 0.62 | 2.37 |
| Hairpin 3.3 | 0.00 | 5.68 | 0.49 | 3.98 | 46.19 | 12.82 | 9.71 | 2.10 | 16.70 | 0.00 | 1.94 | 0.39 | 2.05 |
| Hairpin 3.4 | 0.17 | 7.19 | 0.77 | 3.69 | 45.90 | 11.86 | 10.65 | 1.84 | 15.39 | 0.00 | 1.90 | 0.65 | 2.04 |
| Hairpin 3.5 | 0.00 | 6.45 | 0.48 | 3.26 | 51.76 | 8.13 | 10.04 | 1.51 | 16.08 | 0.00 | 1.92 | 0.36 | 2.85 |
| Hairpin 3.6 | 0.08 | 7.51 | 0.23 | 3.59 | 19.97 | 29.13 | 20.12 | 2.15 | 14.54 | 0.29 | 2.02 | 0.36 | 0.41 |
| Hairpin 3.7 | 0.14 | 7.20 | 0.78 | 2.90 | 26.37 | 24.81 | 17.18 | 1.92 | 15.50 | 0.36 | 2.30 | 0.53 | 0.63 |
| Hairpin 3.8 | 0.11 | 6.34 | 0.46 | 3.23 | 38.58 | 15.25 | 13.54 | 1.89 | 16.91 | 0.00 | 2.36 | 1.34 | 1.34 |
| Hairpin 3.9 | 0.00 | 6.47 | 0.49 | 3.32 | 47.59 | 11.44 | 9.63 | 1.68 | 15.96 | 0.00 | 1.88 | 1.55 | 2.26 |
| Hairpin 3.10 | 0.00 | 6.77 | 0.56 | 3.48 | 53.30 | 7.57 | 9.34 | 1.55 | 15.65 | 0.00 | 1.79 | 0.00 | 3.15 |
| Hairpin 3.11 | 0.00 | 7.05 | 0.59 | 3.61 | 53.62 | 8.87 | 8.36 | 1.55 | 14.35 | 0.00 | 1.99 | 0.00 | 3.11 |
| Hairpin 3.12 | 0.05 | 8.32 | 0.36 | 3.85 | 18.48 | 29.24 | 19.94 | 2.48 | 14.75 | 0.00 | 2.28 | 0.26 | 0.38 |
| Hairpin 4.1 | 0.09 | 6.97 | 0.59 | 3.61 | 53.64 | 8.40 | 8.44 | 1.60 | 15.00 | 0.00 | 1.66 | 0.00 | 3.19 |
| Hairpin 4.2 | 0.07 | 6.81 | 0.22 | 3.27 | 55.06 | 9.16 | 8.71 | 1.26 | 13.63 | 0.19 | 1.33 | 0.30 | 3.08 |
| Hairpin 4.3 | 0.04 | 6.81 | 0.50 | 3.47 | 46.21 | 10.67 | 11.52 | 1.81 | 16.50 | 0.00 | 1.88 | 0.58 | 2.08 |
| Hairpin 5.1 | 0.00 | 8.30 | 0.23 | 3.71 | 17.72 | 28.92 | 20.63 | 2.38 | 14.77 | 0.00 | 2.41 | 0.92 | 0.36 |
| Hairpin 5.2 | 0.19 | 7.15 | 1.55 | 3.56 | 44.58 | 11.44 | 11.59 | 1.77 | 15.67 | 0.00 | 1.84 | 0.65 | 1.94 |
| Hairpin 5.3 | 0.10 | 6.49 | 0.40 | 3.72 | 54.19 | 7.01 | 7.89 | 1.74 | 15.91 | 0.00 | 1.92 | 0.62 | 3.64 |
| Hairpin 5.5 | 0.12 | 6.58 | 0.51 | 3.84 | 54.48 | 6.16 | 7.23 | 1.77 | 16.50 | 0.42 | 1.90 | 0.48 | 4.07 |
| Hairpin 5.6 | 0.00 | 6.67 | 0.50 | 3.66 | 46.32 | 11.56 | 10.48 | 1.83 | 15.99 | 0.00 | 2.15 | 0.84 | 2.10 |
| Hairpin 5.7 | 0.00 | 5.50 | 0.51 | 3.58 | 57.33 | 4.75 | 5.91 | 1.75 | 18.03 | 0.00 | 1.88 | 0.76 | 5.38 |
| Hairpin 5.8 | 0.16 | 6.55 | 1.53 | 3.54 | 48.52 | 9.91 | 8.97 | 1.78 | 16.39 | 0.00 | 1.84 | 0.81 | 2.57 |
| Hairpin 6.1 | 0.10 | 6.35 | 0.57 | 3.48 | 59.00 | 4.77 | 6.26 | 1.48 | 15.95 | 0.00 | 1.80 | 0.25 | 5.35 |
| Hairpin 6.2 | 0.10 | 7.98 | 0.37 | 4.06 | 20.96 | 29.01 | 18.69 | 2.38 | 13.63 | 0.20 | 2.03 | 0.60 | 0.44 |
| Hairpin 6.5 | 0.08 | 6.21 | 0.63 | 3.61 | 60.05 | 5.07 | 5.27 | 1.55 | 15.20 | 0.00 | 1.69 | 0.66 | 5.81 |
| Columbia pBin 19 control | 0.08 | 8.81 | 0.47 | 3.51 | 17.07 | 30.31 | 20.94 | 1.78 | 14.56 | 0.00 | 2.17 | 0.28 | 0.33 |
| Cosuppresion 1.1 | 0.08 | 8.16 | 0.62 | 3.71 | 26.16 | 23.77 | 18.15 | 2.06 | 14.65 | 0.17 | 1.89 | 0.57 | 0.62 |
| Cosuppresion 1.2 | 0.00 | 8.49 | 0.53 | 3.65 | 17.90 | 29.93 | 20.36 | 2.34 | 14.25 | 0.00 | 2.33 | 0.23 | 0.36 |
| Cosuppresion 1.3 | 0.07 | 6.65 | 0.40 | 3.42 | 38.34 | 15.25 | 14.16 | 1.91 | 17.19 | 0.31 | 1.94 | 0.35 | 1.30 |
| Cosuppresion 1.4 | 0.00 | 8.22 | 0.57 | 3.82 | 18.27 | 28.82 | 19.63 | 2.56 | 14.83 | 0.00 | 2.46 | 0.83 | 0.38 |
| Cosuppresion 1.5 | 0.00 | 7.51 | 0.52 | 3.84 | 34.59 | 17.90 | 14.64 | 2.18 | 16.27 | 0.00 | 2.02 | 0.54 | 1.06 |
| Cosuppresion 1.6 | 0.07 | 7.44 | 0.47 | 3.16 | 23.97 | 27.32 | 17.29 | 2.03 | 15.52 | 0.18 | 2.22 | 0.33 | 0.54 |
| Cosuppresion 2.1 | 0.07 | 7.46 | 0.43 | 3.00 | 23.91 | 27.21 | 17.79 | 1.84 | 15.27 | 0.30 | 2.14 | 0.58 | 0.53 |
| Cosuppresion 2.2 | 0.00 | 8.19 | 0.55 | 4.22 | 18.59 | 28.31 | 18.80 | 2.77 | 15.51 | 0.00 | 2.46 | 0.58 | 0.39 |
| Cosuppresion 2.3 | 0.00 | 8.71 | 0.47 | 3.48 | 19.21 | 30.06 | 19.49 | 2.03 | 13.78 | 0.00 | 2.15 | 0.63 | 0.39 |
| Cosuppresion 3.1 | 0.06 | 7.57 | 0.50 | 3.83 | 32.24 | 20.00 | 15.66 | 2.06 | 15.65 | 0.34 | 1.85 | 0.23 | 0.90 |
| Cosuppresion 4.1 | 0.00 | 7.29 | 0.43 | 3.55 | 30.26 | 21.17 | 17.06 | 2.01 | 16.08 | 0.00 | 1.92 | 0.25 | 0.79 |
| Cosuppresion 4.2 | 0.08 | 8.02 | 0.53 | 3.62 | 33.04 | 20.04 | 15.68 | 1.80 | 14.72 | 0.00 | 1.88 | 0.58 | 0.92 |
| Cosuppresion 4.3 | 0.07 | 8.35 | 0.54 | 3.85 | 30.02 | 21.72 | 16.78 | 2.01 | 14.25 | 0.00 | 1.92 | 0.49 | 0.78 |
| Cosuppresion 4.4 | 0.06 | 6.98 | 0.53 | 3.62 | 43.38 | 13.24 | 12.77 | 1.74 | 15.37 | 0.30 | 1.67 | 0.33 | 1.67 |
| Cosuppresion 4.5 | 0.13 | 7.84 | 0.52 | 3.76 | 33.76 | 18.16 | 16.21 | 1.89 | 14.96 | 0.35 | 1.85 | 0.57 | 0.98 |
| Cosuppresion 4.6 | 0.11 | 8.18 | 0.32 | 3.58 | 19.72 | 29.19 | 20.26 | 2.04 | 13.92 | 0.29 | 1.84 | 0.55 | 0.40 |
| Cosuppresion 4.7 | 0.11 | 7.88 | 0.39 | 3.75 | 27.40 | 22.85 | 17.44 | 2.08 | 15.29 | 0.00 | 2.04 | 0.76 | 0.68 |
| Cosuppresion 4.8 | 0.13 | 7.56 | 0.41 | 3.46 | 32.27 | 20.50 | 15.45 | 1.90 | 15.47 | 0.00 | 2.02 | 0.83 | 0.90 |
| Cosuppresion 4.9 | 0.09 | 7.46 | 0.29 | 3.75 | 36.11 | 16.96 | 15.74 | 1.92 | 15.38 | 0.31 | 1.74 | 0.25 | 1.10 |
| Cosuppresion 5.1 | 0.10 | 7.68 | 0.34 | 3.88 | 36.00 | 16.77 | 15.38 | 1.90 | 15.44 | 0.32 | 1.82 | 0.36 | 1.12 |
| Cosuppresion 5.2 | 0.08 | 7.56 | 0.25 | 3.58 | 26.10 | 25.11 | 17.79 | 1.96 | 15.03 | 0.30 | 1.72 | 0.54 | 0.61 |
| Cosuppresion 5.3 | 0.08 | 7.38 | 0.20 | 3.56 | 42.24 | 13.33 | 13.32 | 1.76 | 15.19 | 0.16 | 1.61 | 1.18 | 1.59 |
| Cosuppresion 6.1 | 0.08 | 8.04 | 0.50 | 3.68 | 31.37 | 20.29 | 17.17 | 1.84 | 14.31 | 0.00 | 1.76 | 0.95 | 0.84 |
| Cosuppresion 6.2 | 0.00 | 8.50 | 0.51 | 3.91 | 18.59 | 29.33 | 19.66 | 2.46 | 14.75 | 0.00 | 2.28 | 0.00 | 0.38 |
| Control c24 pGNAP-p450 | 0.07 | 8.30 | 0.10 | 4.78 | 19.68 | 25.91 | 20.56 | 2.97 | 15.29 | 0.31 | 1.79 | 0.24 | 0.42 |

Analysis of the results indicates that transgenic plants harboring a CoP construct (indicated as "hairpin x.x" in the table) have a higher frequency of plants with oil wherein the increase in oleic acid and concomitant decrease in linolenic and linoleic acid is significant than in transgenic plants harboring cosuppression constructs.. Moreover the absolute levels of increase, respectively decrease are higher respectively lower than in transgenic plants harboring cosuppression constructs.

### Example 7. Modifying oil profile using CoP constructs in Brassica.

The T-DNA vector harboring the chimeric gene encoding the CoP construct described in Example 6 is introduced in *Brassica* oilseed rape. Seeds harvested from the transgenic *Brassica sp.* are crashed and oil extracted and the composition of the fatty acids in the oil is analyzed.

Oil from transgenic *Brassica sp.* harboring the CoP construct have significantly increased oleic acid content and decreased linoleic and linolenic acid content. A T-DNA vector harboring a chimeric gene encoding a CoP construct similar to the one described in Example 6, but wherein the sequence of the sense and antisense region corresponding to the Δ12 desaturase encoding ORF is based on a homologous ORF from *Brassica spp.* is constructed and introduced in Brassica oilseed rape.

The sequence of Brassica spp ORFs homologous to Δ12 desaturase encoding ORF from Arabidopsis are available from Genbank database under Accession nrs AF042841 and AF124360.

Seeds harvested from the transgenic *Brassica sp.* are crashed and oil extracted and the composition of the fatty acids in the oil is analyzed. Oil from transgenic *Brassica sp.* harbouring the CoP construct have significantly increased oleic acid content and decreased linoleic and linolenic acid content.

### Example 8. Suppression of an endogenous rust resistance gene in flax.

A CoP construct for suppression of the endogenous rust resistance gene was made consisting of
1. a CaMV35S promoter; operably linked to
2. part of an endogenous rust resistance gene (n) from flax (about 1500 bp long) in the sense orientation; ligated to
3. a similar part of the endogenous rust resistance gene from flax (about 1450 bp long) in antisense orientation so that a perfect inverted repeat without spacer sequence is generated wherein each repeat is about 1450 bp long; followed by
4. a nos terminator.

Plain antisense constructs were made using a similar fragment as described sub 3 above inserted between a CaMV35S promoter and a nos terminator.

Flax plants containing the n gene (which gives resistance to a strain of flax rust) were transformed by these CoP and antisense constructs. If suppression occurs, the plants become susceptible to the rust strain. If the construct has no effect, the transformed plants remain resistant to the rust strain.

### Results

| | |
|---|---|
| ngc-b sense/antisense | 3 suppressed out of 7 |
| ngc-b antisense | 0 suppressed out of 12 |

While the invention has been described and illustrated herein by references to various specific material, procedures and examples, it is understood that the invention is not restricted to the particular material, combinations of material, and procedures selected for that purpose. Numerous variations of such details can be implied and will be appreciated by those skilled in the art.

### REFERENCES

An et al. (1996) The Plant Cell 8, 15-30
Barry et al. (1993) Proc Natl Acad Sci 90, 4557-4561
Baulcombe (1996) Plant Cell 8, 1833-1844
Braun and Hemenway (1992) Plant Cell 4, 735-744
Brederode et al. (1995) Virology 207, 467-474
Carr et al. (1992) Mol. Plant-Microb. Interact. 5, 397-404
Christensen and Quail (1996) Transgenic Research 5, 213-218
Croy Plant Molecular Biology Labfax (1993) BIOS Scientific Publications Ltd (UK) and Blackwell Scientific Publications, UK.
de Carvalho Niebel et al. (1995) Plant Cell 7, 347-358
English et al. (1996) Plant Cell 8, 179-188
Fire et al. (1998) Nature 391, 806-811
Fromm et al. (1990) Bio/Technology 8: 833
Gleave, (1992) Plant Mol. Biol. 20: 1203-1207
Goodwin et al. (1996) Plant Cell 8, 95-105
Gordon-Kamm et al. (1990) The Plant Cell 2: 603
Harpster et al. (1988) Mol. Gen. Genet. 212, 182-190
Hobbs et al. (1990) Plant Mol. Biol. 15, 851-864
Hudspeth et al. (1989) Plant Mol Biol 12: 579-589
Ingelbrecht et al. (1994) Proc. Natl. Acad. Sci. USA 91, 10502-10506
Jefferson et al. (1987) EMBO J. 6, 3901-3907
Kawchuck et al. (1991) Molecular plant-microbe interactions 4, 247-253.
Keil et al. (1989) EMBO J. 8: 1323-1330
Keller et al. (1988) EMBO J. 7: 3625-3633
Keller et al. (1989) Genes Devel. 3: 1639-1646
Landsman et al. (1988) Mol Gen Genet 214, 68-73
Lindbo & Dougherty (1992a) Mol. Plant Micr. Int 5, 144-153
Lindbo & Dougherty (1992b) Virology 189, 725-733
Lindbo et al. (1993) Plant Cell 5, 1749-1759
Longstaff et al. (1993) EMBO J. 12, 379-386
MacDonald et al. (1991) Nucl. Acids Res. 19, 5575-5581
Metzlaff et al. (1997) Cell 88, 845-854
Meyer et al. (1987) Nature 330: 677
Mueller et al. (1995) Plant J. 7, 1001-1003
Ohta et al. (1990) Plant Cell. Physiol. 31, 805-813
Pang et al. (1996) Plant J. 9, 899-909
Peleman et al. 1989 Gene 84: 359-369
Powell-Abel et al. (1986) Science 232, 738-743
Powell et al. (1990) Virology 175, 124-130
Que et al. (1998) The Plant Journal 13, 401-409
Sambrook et al. (1989) Molecular Cloning: A Laboratory Manual, Second Edition, Cold Spring Harbor Laboratory Press, NY
Sanford and Johnston, (1985) J. Theor. Biol. 113, 395-405
Schiebel et al. (1993a) Journal of Biological Chemistry 268: 11851-11857
Schiebel et al. (1993b) Journal of Biological Chemistry 268: 11858-11867
Smith et al. (1994) Plant Cell 6, 1441-1453
Stalberg et al., Plant Molecular Biol. 23, 671-683
Stam et al. (1997) Ann. Botan. 79, 3-12
Wagner and Sun (1998) Nature 391, 744-745
Wang et al. (1997) Journal of Genetics and Breeding 3
Wilbur and Lipmann (1983) Proc. Nat. Acad. Sci. U.S.A. 80: 726
Zheng et al. (1991) Plant Physiol. 97, 832-835
Zuker and Stiegler (1981) Nucl. Acids Res. 9, 133-148

## Claims

1. A method for reducing the phenotypic expression of a nucleic acid of interest, which can be expressed in a eucaryotic cell, comprising the step of introducing into said cell a chimeric DNA comprising the following operably linked parts: a) a promoter, operative in said eucaryotic cell;
b) a DNA region, which when transcribed, yields an RNA molecule with a nucleotide sequence comprising
i. a sense nucleotide sequence including at least 15 consecutive nucleotides having 100% sequence identity with at least part of a coding region of said nucleic acid of interest; and
ii. an antisense nucleotide sequence including at least 15 consecutive nucleotides, having 100% sequence identity with the complement of said at least 15 consecutive nucleotides of said sense nucleotide sequence;
wherein the RNA is capable of forming an artificial hairpin RNA structure with a double stranded RNA stem by base-pairing between the regions with sense and antisense nucleotide sequence such that at least said 15 consecutive nucleotides of the sense sequence basepair with said 15 consecutive nucleotides of the antisense sequence; wherein said nucleic acid of interest is a gene integrated in the genome of said eucaryotic cell,
and wherein said method is not a method of treatment of the human or animal body by surgery or therapy, or a diagnostic method practised on the human or animal body.

2. A method according to claim 1 wherein the chimeric DNA further comprises a DNA region involved in transcription termination and polyadenylation.

3. The method of claim 1 or 2, wherein said RNA molecule further comprises a spacer nucleotide sequence located between said sense and said antisense nucleotide sequence.

4. The method of claim 1 or 2 wherein the sense nucleotide sequence includes a sequence of 20 nucleotides with 100% sequence identity to the corresponding part of the nucleic acid of interest, and wherein the antisense nucleotide sequence includes a sequence of 20 nucleotides with 100% sequence identity to the complement of the sense nucleotide sequence.

5. The method of any one of claims 1 or 2 wherein said antisense nucleotide sequence has a total length corresponding to that of the sense nucleotide sequence.

6. The method of any one of claims 1 or 2 wherein the antisense nucleotide sequence differs in length from the sense nucleotide sequence by about 10%.

7. The method of claim 1 wherein the RNA molecule consists of the hairpin RNA.

8. The method of claim 1, wherein said gene is an endogenous gene.

9. The method of claim 1, wherein said gene is a foreign transgene.

10. The method of claim 1 , wherein said nucleic acid of interest is comprised in the genome of an infecting virus.

11. The method of claim 10, wherein said infecting virus is an RNA virus.

12. The method according to claim 1, or 2, wherein said eucaryotic cell is a plant cell.

13. The method of claim 12, wherein said plant cell is comprised within a plant.

14. Method according to claim 13 wherein the plant is a crop plant or a vegetable plant.

15. Method according to claim 14 wherein the plant is corn, rice, wheat, barley, sugarcane, cotton, oilseed rape, soybean, chicory, brassica vegetables, lettuce, tomato, tobacco, potato, or sugarbeet.

16. Method according to any one of claims 1 to 15, wherein the reduced phenotypic expression is used to obtain shatter resistance, to obtain modified flower colour patterns, to obtain nematode resistant plants, to delay fruit ripening, to obtain male sterility, to reduce the presence of secondary metabolites in plants, to modify the profile of metabolites synthesized in a plant cell by metabolic engineering, to delay senescence, to alter lignification, to alter the fibre quality in cotton, to increase bruising resistance in potatoes by reducing polyphenoloxidase, to obtain virus resistance, or to obtain disease or pest resistance.

17. A eucaryotic cell comprising a nucleic acid of interest, which can be phenotypically expressed, further comprising a chimeric DNA molecule comprising the following operably linked parts:
a) a promoter, operative in said eucaryotic cell;
b) a DNA region, which when transcribed, yields an RNA molecule with at least one RNA region with a nucleotide sequence comprising
i) a sense nucleotide sequence including at least 15 consecutive nucleotides having 100% sequence identity with at least part of a coding region of the nucleic acid of interest; and
ii) an antisense nucleotide sequence including at least 15 consecutive nucleotides, having 100% sequence identity with the complement of said at least 15 consecutive nucleotides of said sense nucleotide sequence;
wherein the RNA is capable of forming an artificial hairpin RNA structure with a double stranded RNA stem by base-pairing between the regions with sense and antisense nucleotide sequence, such that at least said 15 consecutive nucleotides of the sense sequence base pair with said 15 consecutive nucleotides of the antisense sequence and
wherein said nucleic acid of interest is a gene integrated in the genome of said eucaryotic cell.

18. A cell according to claim 17 wherein the chimeric DNA molecule further comprises a DNA region involved in transcription termination and polyadenylation.

19. The eucaryotic cell of claim 18, which is a plant cell.

20. A plant comprising the plant cell of claim 19.

21. The cell according to claim 17 wherein the RNA molecule further comprises a spacer nucleotide sequence located between said sense and said antisense nucleotide sequence.

22. The cell according to claim 17 wherein the sense nucleotide sequence includes a sequence of 20 nucleotides with 100% sequence identity to the corresponding part of the nucleic acid of interest, and wherein the antisense nucleotide sequence includes a sequence of 20 nucleotides with 100% sequence identity to the complement of the sense nucleotide sequence.

23. The cell according to claim 17 wherein said antisense nucleotide sequence has a total length corresponding to that of the sense nucleotide sequence.

24. The cell according to claim 17 wherein the antisense nucleotide sequence differs in length from the sense nucleotide sequence by about 10%.

25. The cell according to claim 17 , for use in reducing viral infection, or in obtaining resistance to a disease.

## Patentansprüche

1. Verfahren zur Reduzierung der phänotypischen Expression einer Nukleinsäure von Interesse, die in einer eukaryotischen Zelle exprimiert werden kann, umfassend die Einführung einer Chimären DNA, die die folgenden funktionell verknüpften Teile umfasst, in die Zelle:
a) einen Promotor, der in der eukaryotischen Zelle funktionsfähig ist;
b) eine DNA-Region, die bei Transkription, ein RNA-Molekül mit einer Nukleotidsequenz ergibt, umfassend:
i. eine Sense-Nukleotidsequenz, die mindestens 15 aufeinanderfolgende Nukleotide beinhaltet, die eine 100 %-ige Sequenzidentität mit mindestens einem Teil einer codierenden Region der Nukleinsäure von Interesse aufweist; und
ii. eine Antisense-Nukleotidsequenz, die mindestens 15 aufeinanderfolgenden Nukleotiden beinhaltet, die eine 100 %-ige Sequenzidentität mit dem Komplement der mindestens 15 aufeinanderfolgenden Nukleotide der Sense-Nukleotidsequenz aufweist;
wobei die RNA in der Lage ist, eine künstliche Haarnadel-RNA-Struktur mit einem doppelsträngigen RNA-Stamm durch Basenpaarung zwischen den Regionen mit Sense- und Antisense-Nukleotidsequenz derart auszubilden, dass mindestens die 15 aufeinanderfolgenden Nukleotide der Sense-Sequenz mit den 15 aufeinanderfolgenden Nukleotiden der Antisense-Sequenz eine Basenpaarung eingehen;
wobei die Nukleinsäure von Interesse ein Gen ist, das in das Genom der eukaryotischen Zelle integriert ist, und wobei das Verfahren kein Verfahren für eine Behandlung des menschlichen oder tierischen Körpers durch Operation oder Therapie oder ein diagnostisches Verfahren ist, das am menschlichen oder tierischen Körper praktiziert wird;

2. Verfahren nach Anspruch 1, wobei die Chimäre DNA ferner eine DNA-Region umfasst, die an der Transkriptionsterminierung und Polyadenylierung beteiligt ist.

3. Verfahren nach Anspruch 1 oder 2, wobei das RNA-Molekül ferner eine Spacer-Nukleotidsequenz umfasst, die sich zwischen der Sense- und der Antisense-Nukleotidsequenz befindet.

4. Verfahren nach Anspruch 1 oder 2, wobei die Sense-Nukleotidsequenz eine Sequenz von 20 Nukleotiden mit einer 100 %-iger Sequenzidentität zu dem entsprechenden Teil der Nukleinsäure von Interesse beinhaltet und wobei die Antisense-Nukleotidsequenz eine Sequenz von 20 Nukleotiden mit einer 100 %-iger Sequenzidentität zum Komplement der Sense-Nukleotidsequenz beinhaltet.

5. Verfahren nach einem der Ansprüche 1 oder 2, wobei die Antisense-Nukleotidsequenz eine Gesamtlänge aufweist, die der der Sense-Nukleotidsequenz entspricht.

6. Verfahren nach einem der Ansprüche 1 oder 2, wobei sich die Antisense-Nukleotidsequenz in der Länge von der Sense-Nukleotidsequenz um etwa 10 % unterscheidet.

7. Verfahren nach Anspruch 1, wobei das RNA-Molekül aus der Haarnadel-RNA besteht.

8. Verfahren nach Anspruch 1, wobei das Gen ein endogenes Gen ist.

9. Verfahren nach Anspruch 1, wobei das Gen ein fremdes Transgen ist.

10. Verfahren nach Anspruch 1, wobei die Nukleinsäure von Interesse in dem Genom eines infizierenden Virus enthalten ist.

11. Verfahren nach Anspruch 10, wobei das infizierende Virus ein RNA-Virus ist.

12. Verfahren nach Anspruch 1 oder 2, wobei die eukaryotische Zelle eine Pflanzenzelle ist.

13. Verfahren nach Anspruch 12, wobei die Pflanzenzelle innerhalb einer Pflanze enthalten ist.

14. Verfahren nach Anspruch 13, wobei die Pflanze eine Nutzpflanze oder eine Gemüsepflanze ist.

15. Verfahren nach Anspruch 14, wobei die Pflanze Mais, Reis, Weizen, Gerste, Zuckerrohr, Baumwolle, Raps, Sojabohnen, Zichorie, Brassica-Gemüse, Salat, Tomate, Tabak, Kartoffel oder Zuckerrübe ist.

16. Verfahren nach einem der Ansprüche 1 bis 15, wobei die verringerte phänotypische Expression verwendet wird, um eine Bruchresistenz zu erhalten, modifizierte Blütenfarbmuster zu erhalten, nematodenresistente Pflanzen zu erhalten, um die Fruchtreife zu verzögern, um männliche Sterilität zu erhalten, um das Vorhandensein von Sekundärmetaboliten in Pflanzen zu verringern, um das Profil von Metaboliten zu modifizieren, die in einer Pflanzenzelle durch metabolisches Engineering synthetisiert wurden, um die Seneszenz zu verzögern, um die Lignifizierung zu verändern, um die Faserqualität in Baumwolle zu verändern, um die Resistenz gegen Druckstellen in Kartoffeln durch Verringern der Polyphenoloxidase zu erhöhen, um Virenresistenz zu erhalten oder um Krankheits- oder Schädlingsresistenz zu erhalten.

17. Eukaryotische Zelle, die eine Nukleinsäure von Interesse enthält, die phänotypisch exprimiert werden kann, ferner umfassend ein chimäres DNA-Molekül, das die folgenden funktionsfähig verknüpften Teile umfasst:
a) einen Promotor, der in der eukaryotischen Zelle wirksam ist;
b) eine DNA-Region, die bei Transkription ein RNA-Molekül mit mindestens einer RNA-Region mit einer Nukleotidsequenz umfassend folgende Komponente, ergibt:
i. eine Sense-Nukleotidsequenz, die mindestens 15 aufeinanderfolgende Nukleotide beinhaltet, die 100 %-ige Sequenzidentität mit mindestens einem Teil einer codierenden Region der Nukleinsäure von Interesse aufweist; und
ii. eine Antisense-Nukleotidsequenz, die mindestens 15 aufeinanderfolgenden Nukleotiden beinhaltet, die eine 100 %-ige Sequenzidentität mit dem Komplement der mindestens 15 aufeinanderfolgenden Nukleotide der Sense-Nukleotidsequenz aufweist;
wobei die RNA in der Lage ist, eine künstliche Haarnadel-RNA-Struktur mit einem doppelsträngigen RNA-Stamm durch Basenpaarung zwischen den Regionen mit Sense- und Antisense-Nukleotidsequenz derart auszubilden, dass mindestens die 15 aufeinanderfolgenden Nukleotide der Sense-Sequenz mit den 15 aufeinanderfolgenden Nukleotiden der Antisense-Sequenz eine Basenpaarung eingehen und wobei die Nukleinsäure von Interesse ein Gen ist, das in das Genom der eukaryotischen Zelle integriert ist.

18. Zelle nach Anspruch 17, wobei das chimäre DNA-Molekül ferner eine DNA-Region umfasst, die an der Transkriptionstermination und Polyadenylierung beteiligt ist.

19. Eukaryotische Zelle nach Anspruch 18, die eine Pflanzenzelle ist.

20. Pflanze, umfassend die Pflanzenzelle nach Anspruch 19.

21. Zelle nach Anspruch 17, wobei das RNA-Molekül ferner eine Spacer-Nukleotidsequenz umfasst, die sich zwischen der Sense- und der Antisense-Nukleotidsequenz befindet.

22. Zelle nach Anspruch 17, wobei die Sense-Nukleotidsequenz eine Sequenz von 20 Nukleotiden mit 100 %-iger Sequenzidentität zu dem entsprechenden Teil der Nukleinsäure von Interesse beinhaltet und wobei die Antisense-Nukleotidsequenz eine Sequenz von 20 Nukleotiden mit 100 %-iger Sequenzidentität zu dem Komplement der Sense-Nukleotidsequenz beinhaltet.

23. Zelle nach Anspruch 17, wobei die Antisense-Nukleotidsequenz eine Gesamtlänge aufweist, die der der Sense-Nukleotidsequenz entspricht.

24. Zelle nach Anspruch 17, wobei sich die Antisense-Nukleotidsequenz in der Länge von der Sense-Nukleotidsequenz um etwa 10 % unterscheidet.

25. Zelle nach Anspruch 17 zur Verwendung beim Verringern der Virusinfektion oder zum Erlangen einer Resistenz gegen eine Krankheit.

## Revendications

1. Méthode pour réduire l'expression phénotypique d'un acide nucléique d'intérêt, qui peut être exprimé dans une cellule eucaryote, comprenant l'étape d'introduction, dans ladite cellule, d'un ADN chimérique comprenant les parties suivantes liées de manière fonctionnelle :
a) un promoteur fonctionnel dans ladite cellule eucaryote ;
b) une région d'ADN qui, lorsqu'elle est transcrite, produit une molécule d'ARN comportant une séquence nucléotidique comprenant
i. une séquence nucléotidique sens comprenant au moins 15 nucléotides consécutifs ayant une identité de séquence de 100 % avec au moins une partie d'une région codante dudit acide nucléique d'intérêt ; et
ii. une séquence nucléotidique antisens comprenant au moins 15 nucléotides consécutifs, ayant une identité de séquence de 100 % avec le complément desdits au moins 15 nucléotides consécutifs de ladite séquence nucléotidique sens ;
dans laquelle l'ARN est capable de former, par appariement des bases entre les régions ayant une séquence nucléotidique sens et antisens, une structure artificielle d'ARN en épingle à cheveux avec une tige d'ARN double brin de telle sorte qu'au moins lesdits 15 nucléotides consécutifs de la séquence sens s'apparient avec lesdits 15 nucléotides consécutifs de la séquence antisens ;
dans laquelle ledit acide nucléique d'intérêt est un gène intégré dans le génome de ladite cellule eucaryote, et dans laquelle ladite méthode n'est pas une méthode de traitement du corps humain ou animal par chirurgie ou thérapie, ou une méthode de diagnostic appliquée au corps humain ou animal.

2. Méthode selon la revendication 1, dans laquelle l'ADN chimérique comprend en outre une région ADN impliquée dans la terminaison de la transcription et la polyadénylation.

3. Méthode selon la revendication 1 ou 2, dans laquelle ladite molécule d'ARN comprend en outre une séquence nucléotidique d'espacement située entre ladite séquence nucléotidique sens et ladite séquence nucléotidique antisens.

4. Méthode selon la revendication 1 ou 2, dans laquelle la séquence nucléotidique sens comporte une séquence de 20 nucléotides ayant une identité de séquence de 100 % avec la partie correspondante de l'acide nucléique d'intérêt, et dans lequel la séquence nucléotidique antisens comporte une séquence de 20 nucléotides ayant une identité de séquence de 100 % avec le complément de la séquence nucléotidique sens.

5. Méthode selon l'une quelconque des revendications 1 ou 2, dans laquelle ladite séquence nucléotidique antisens présente une longueur totale correspondant à celle de la séquence nucléotidique sens.

6. Méthode selon l'une quelconque des revendications 1 ou 2, dans laquelle la séquence nucléotidique antisens diffère en longueur de la séquence nucléotidique sens d'environ 10 %.

7. Méthode selon la revendication 1, dans laquelle la molécule d'ARN consiste en l'ARN en épingle à cheveux.

8. Méthode selon la revendication 1, dans laquelle ledit gène est un gène endogène.

9. Méthode selon la revendication 1, dans laquelle ledit gène est un transgène étranger.

10. Méthode selon la revendication 1, dans laquelle ledit acide nucléique d'intérêt est compris dans le génome d'un virus infectant.

11. Méthode selon la revendication 10, dans laquelle ledit virus infectant est un virus à ARN.

12. Méthode selon la revendication 1 ou 2, dans laquelle ladite cellule eucaryote est une cellule végétale.

13. Méthode selon la revendication 12, dans laquelle ladite cellule végétale est comprise dans une plante.

14. Méthode selon la revendication 13, dans laquelle la plante est une plante cultivée ou un plant de légume.

15. Méthode selon la revendication 14, dans laquelle la plante est le maïs, le riz, le blé, l'orge, la canne à sucre, le coton, le colza, le soja, la chicorée, les légumes brassica, la laitue, la tomate, le tabac, la pomme de terre ou la betterave à sucre.

16. Méthode selon l'une quelconque des revendications 1 à 15, dans laquelle l'expression phénotypique réduite est utilisée pour obtenir une résistance à l'éclatement, pour obtenir des motifs de couleur de fleur modifiés, pour obtenir des plantes résistantes aux nématodes, pour retarder la maturation des fruits, pour obtenir une stérilité mâle, pour réduire la présence de métabolites secondaires dans les plantes, pour modifier le profil des métabolites synthétisés dans une cellule végétale par l'ingénierie métabolique, pour retarder la sénescence, pour modifier la lignification, pour modifier la qualité des fibres dans le coton, pour augmenter la résistance aux meurtrissures des pommes de terre en réduisant la polyphénoloxidase, pour obtenir une résistance aux virus ou pour obtenir une résistance à une maladie ou à un ravageur.

17. Cellule eucaryote comprenant un acide nucléique d'intérêt, qui peut être exprimé phénotypiquement, comprenant en outre une molécule d'ADN chimérique comprenant les parties suivantes liées de manière fonctionnelle :
a) un promoteur fonctionnel dans ladite cellule eucaryote ;
b) une région ADN qui, lorsqu'elle est transcrite, produit une molécule d'ARN ayant au moins une région d'ARN ayant une séquence nucléotidique comprenant
i. une séquence nucléotidique sens comprenant au moins 15 nucléotides consécutifs ayant une identité de séquence de 100 % avec au moins une partie d'une région codante de l'acide nucléique d'intérêt ; et
ii. une séquence nucléotidique antisens comportant au moins 15 nucléotides consécutifs, ayant une identité de séquence de 100 % avec le complément desdits au moins 15 nucléotides consécutifs de ladite séquence nucléotidique sens ;
dans laquelle l'ARN est capable, par appariement des bases entre les régions ayant la séquence nucléotidique sens et antisens, de former une structure artificielle d'ARN en épingle à cheveux avec une tige d'ARN double brin, de telle sorte qu'au moins lesdits 15 nucléotides consécutifs de la séquence sens s'apparient avec lesdits 15 nucléotides consécutifs de la séquence antisens, et dans laquelle ledit acide nucléique d'intérêt est un gène intégré au génome de ladite cellule eucaryote.

18. Cellule selon la revendication 17, dans laquelle la molécule d'ADN chimérique comprend en outre une région ADN impliquée dans la terminaison de la transcription et la polyadénylation.

19. Cellule eucaryote selon la revendication 18, qui est une cellule végétale.

20. Plante comprenant la cellule végétale de la revendication 19.

21. Cellule selon la revendication 17, dans laquelle la molécule d'ARN comprend en outre une séquence nucléotidique d'espacement située entre ladite séquence nucléotidique sens et antisens.

22. Cellule selon la revendication 17, dans laquelle la séquence nucléotidique sens comporte une séquence de 20 nucléotides avec une identité de séquence de 100 % avec la partie correspondante de l'acide nucléique d'intérêt, et dans laquelle la séquence nucléotidique antisens comporte une séquence de 20 nucléotides avec 100 % identité de séquence avec le complément de la séquence nucléotidique sens.

23. Cellule selon la revendication 17, dans laquelle ladite séquence nucléotidique antisens présente une longueur totale correspondant à celle de la séquence nucléotidique sens.

24. Cellule selon la revendication 17, dans laquelle la séquence nucléotidique antisens diffère en longueur de la séquence nucléotidique sens d'environ 10 %.

25. Cellule selon la revendication 17, destinée à être utilisée pour réduire l'infection virale ou pour obtenir une résistance à une maladie.


### SEQUENCE LISTING

<110> Waterhouse, Peter M
   Wang, Ming-Bo
   Graham, Michael W
   Commonwealth Scientific and Industrial Research Or
<120> Methods and means for obtaining modified phenotypes
<130> echidna
<140>
   <141>
<150> US SN 09/056,767
   <151> 1998-03-08
<150> US SN 09/127735
   <151> 1998-08-03
<160> 7
<170> PatentIn Ver. 2.0
<210> 1
   <211> 854
   <212> DNA
   <213> Potato virus Y
<220>
   <223> fragment of the NIa ORF
<400> 1
   aagctttgaa gattgatttg atgccacata acccactcaa aatttgtgac aaaacaaatg 60
   gcattgccaa atttcctgag agagagttcg agctaaggca gactgggcca gctgtagaag 120
   tcgacgtgaa ggacatacca gcacaggagg tggaacatga agctaaatcg ctcatgagag 180
   gcttgagaga cttcaaccca attgcccaaa cagtttgtag gctgaaagta tctgttgaat 240
   atgggacatc agagatgtac ggttttggat ttggagcgta cataatagcg aaccaccatt 300
   tgttcaggag ttataatggt tccatggagg tacgatccat gcacggtaca ttcagggtaa 360
   agaatctaca cagtttgagc gttctgccaa ttaaaggtag ggacatcatc ctcattaaaa 420
   tgccaaaaga tttccctgtc tttccacaga aattgcattt ccgagctcct acacagaacg 480
   aaagaatttg tttagttgga accaactttc aggagaagta tgcatcgtcg atcatcacag 540
   aagcaagcac tacttacaat ataccaggca gcacattctg gaagcattgg attgaaacag 600
   ataatggaca ctgtggacta ccagtggtga gcactgccga tggatgtcta gtcggaattc 660
   acagtttggc aaacaatgca cacaccacga actactactc agccttcgat gaagattttg 720
   aaagcaagta cctccgaacc aatgagcaca atgaatgggt caagtcttgg atttataatc 780
   cagacacagt gttgtggggc ccgttgaaac ttaaagacag cactcctaaa gggttattta 840
   aaacaacaaa gctt                                                   854
<210> 2
   <211> 2186
   <212> DNA
   <213> Artificial Sequence
<220>
   <223> Description of Artificial Sequence:coding region of the Gusd CoP construct
<220>
   <221> misc_structure
   <222>( ) .. )
   <223> deficient Gus coding region
<220>
   <221> misc_feature
   <222> () .. (2186)
   <223> antisense to the 5' end of the Gus coding region
<400> 2
   atggtacgtc ctgtagaaac cccaacccgt gaaatcaaaa aactcgacgg cctgtgggca 60
   ttcagtctgg atcgcgaaaa ctgtggaatt gatcagcgtt ggtgggaaag cgcgttacaa 120
   gaaagccggg caattgctgt gccaggcagt tttaacgatc agttcgccga tgcagatatt 180
   cgtaattatg cgggcaacgt ctggtatcag cgcgaagtct ttataccgaa aggttgggca 240
   ggccagcgta tcgtgctgcg tttcgatgcg gtcactcatt acggcaaagt gtgggtcaat 300
   aatcaggaag tgatggagca tcagggcggc tatacgccat ttgaagccga tgtcacgccg 360
   tatgttattg ccgggaaaag tgtacgtatc accgtttgtg tgaacaacga actgaactgg 420
   cagactatcc cgccgggaat ggtgattacc gacgaaaacg gcaagaaaaa gcagtcttac 480
   ttccatgatt tctttaacta tgccggaatc catcgcagcg taatgctcta caccacgccg 540
   aacacctggg tggacgatat ctacccgctt cgcgtcggca tccggtcagt ggcagtgaag 600
   ggcgaacagt tcctgattaa ccacaaaccg ttctacttta ctggctttgg tcgtcatgaa 660
   gatgcggact tgcgtggcaa aggattcgat aacgtgctga tggtgcacga ccacgcatta 720
   atggactgga ttggggccaa ctcctaccgt acctcgcatt acccttacgc tgaagagatg 780
   ctcgactggg cagatgaaca tggcatcgtg gtgattgatg aaactgctgc tgtcggcttt 840
   aacctctctt taggcattgg tttcgaagcg ggcaacaagc cgaaagaact gtacagcgaa 900
   gaggcagtca acggggaaac tcagcaagcg cacttacagg cgattaaaga gctgatagcg 960
   cgtgacaaaa accacccaag cgtggtgatg tggagtattg ccaacgaacc ggatacccgt 1020
   ccgcaaggtg cacgggaata tttcgcgcca ctggcggaag caacgcgtaa actcgacccg 1080
   acgcgtccga tcacctgcgt caatgtaatg ttctgcgacg ctcacaccga taccatcagc 1140
   gatctctttg atgtgctgtg cctgaaccgt tattacggat ggtatgtcca aagcggcgat 1200
   ttggaaacgg cagagaaggt actggaaaaa gaacttctgg cctggcagga gaaactgcat 1260
   cagccgatta tcatcaccga atacggcgtg gatacgttag ccgggctgca ctcaatgtac 1320
   accgacatgt ggagtgaaga gtatcagtgt gcatggctgg atatgtatca ccgcgtcttt 1380
   gatcgcgtca gcgccgtcgt cggtgaacag gtatggaatt tcgccgattt tgcgacctcg 1440
   caaggcatat tgcgcgttgg cggtaacaag aaagggatct tcactcgcga ccgcaaaccg 1500
   aagtcggcgg cttttctgct gcaaaaacgc tggactggca tgaacttcgg tgaaaaaccg 1560
   cagcagggag gcaaacaatg aaacagacgc gtggttacag tcttgcgcga catgcgtcac 1620
   cacggtgata tcgtccaccc aggtgttcgg cgtggtgtag agcatacgct gcgatggatt 1680
   ccggcatagt taaagaaatc atggaagtaa gactgctttt tcttgccgtt ttcgtcggta 1740
   atcaccattc ccggcgggat agtctgccag ttcagttcgt tgttcacaca aacggtgata 1800
   cgtacacttt tcccggcaat aacatacggc gtgacatcgg cttcaaatgg cgtatagccg 1860
   ccctgatgct ccatcacttc ctgattattg acccacactt tgccgtaatg agtgaccgca 1920
   tcgaaacgca gcacgatacg ctggcctgcc caacctttcg gtataaagac ttcgcgctga 1980
   taccagacgt tgcccgcata attacgaata tctgcatcgg cgaactgatc gttaaaactg 2040
   cctggcacag caattgcccg gctttcttgt aacgcgcttt cccaccaacg ctgatcaatt 2100
   ccacagtttt cgcgatccag actgaatgcc cacaggccgt cgagtttttt gatttcacgg 2160
   gttggggttt ctacaggacg taccat                                      2186
<210> 3
   <211> 208
   <212> DNA
   <213> Artificial Sequence
<220>
<223> Description of Artificial Sequence:5'UTR of Johnson mosaic virus
<400> 3
   cgccccgggc ccaacacaac acaacagaac ctacgtcaat tgattttatc aatcgcaaag 60
   ccttacaaag atcttcgcag tcgttcatca acagattcac cgaaccattc ttgttagctc 120
   tcgcacagag ataagcagga aaccatggca ggtgagtgga acacagtttg atagtaagag 180
   aaaccagagg aagactgcag gtacccgc                                    208
<210> 4
   <211> 1150
   <212> DNA
   <213> Artificial Sequence
<220>
   <223> Description of Artificial Sequence:Subterannean clover virus S4 promoter with S7 enhancer
<400> 4
   aatctgcagc ggccgcttaa tagtaattat gattaattat gagataagag ttgttattat 60
   gcttatgagg aataaagaat gattaatatt gtttaatttt attccgcgaa gcggtgtgtt 120
   atgtttttgt tggagacatc acgtgactct cacgtgatgt ctccgcgaca ggctggcacg 180
   gggcttagta ttaccccgtg ccggatcaga gacatttgac taaatattga cttggaataa 240
   tagcccttgg attagatgac acgtggacgc tcaggatctg tgatgctagt gaagcgctta 300
   agctgaacga atctgacgga agagcggaca tacgcacatg gattatggcc cacatgtcta 360
   aagtgtatct ctttacagct atattgatgt gacgtaagat gctttacttc gcttcgaagt 420
   aaagtaggaa attgctcgct aagttattct tttctgaaag aaattattta attctaatta 480
   aattaaatga gtcgctataa atagtgtcga tgctgcctca catcgtattc ttcttcgcat 540
   cgtctgttct ggttttaagc gggatccagg cctcgagata tcggtacctt gttattatca 600
   ataaaagaat ttttattgtt attgtgttat ttggtaattt atgcttataa gtaattctat 660
   gattaattgt gaattattaa gactaatgag gataataatt gaatttgatt aaattaactc 720
   tgcgaagcta tatgtctttc acgtgagagt cacgtgatgt ctccgcgaca ggctggcacg 780
   gggcttagta ttaccccgtg ccgggatcag agacatttga ctaaatgttg acttggaata 840
   atagcccttg gattagatga cacgtggacg ctcaggatct gtgatgctag tgaagcgctt 900
   aagctgaacg aatctgacgg aagagcggac aaacgcacat ggactatggc ccactgcttt 960
   attaaagaag tgaatgacag ctgtctttgc ttcaagacga agtaaagaat agtggaaaac 1020
   gcgtaaagaa taagcgtact cagtacgctt cgtggcttta tataaatagt gcttcgtctt 1080
   attcttcgtt gtatcatcaa cgaagaagtt aagctttgtt ctgcgtttta atgatcgatg 1140
   gccagtcgac 1150
<210> 5
   <211> 1052
   <212> DNA
   <213> Artificial Sequence
<220>
   <223> Description of Artificial Sequence: subterranean clover virus promoter S4 with S4 enhancer
<400> 5
   ggatccaggc ctcgagatat cggtaccttg ttattatcaa taaaagaatt tttattgtta 60
   ttgtgttatt tggtaattta tgcttataag taattctatg attaattgtg aattattaag 120
   actaatgagg ataataattg aatttgatta aattaactct gcgaagctat atgtctttca 180
   cgtgagagtc acgtgatgtc tccgcgacag gctggcacgg ggcttagtat taccccgtgc 240
   cgggatcaga gacatttgac taaatgttga cttggaataa tagcccttgg attagatgac 300
   acgtggacgc tcaggatctg tgatgctagt gaagcgctta agctgaacga atctgacgga 360
   agagcggaca aacgcacatg gactatggcc cactgcttta ttaaagaagt gaatgacagc 420
   tgtctttgct tcaagacgaa gtaaagaata gtggaaaacg cgtggatcca ggcctcgaga 480
   tatcggtacc ttgttattat caataaaaga atttttattg ttattgtgtt atttggtaat 540
   ttatgcttat aagtaattct atgattaatt gtgaattatt aagactaatg aggataataa 600
   ttgaatttga ttaaattaac tctgcgaagc tatatgtctt tcacgtgaga gtcacgtgat 660
   gtctccgcga caggctggca cggggcttag tattaccccg tgccgggatc agagacattt 720
   gactaaatgt tgacttggaa taatagccct tggattagat gacacgtgga cgctcaggat 780
   ctgtgatgct agtgaagcgc ttaagctgaa cgaatctgac ggaagagcgg acaaacgcac 840
   atggactatg gcccactgct ttattaaaga agtgaatgac agctgtcttt gcttcaagac 900
   gaagtaaaga atagtggaaa acgcgtaaag aataagcgta ctcagtacgc ttcgtggctt 960
   tatataaata gtgcttcgtc ttattcttcg ttgtatcatc aacgaagaag ttaagctttg 1020
   ttctgcgttt taatgatcga tggccagtcg ac                                1052
<210> 6
   <211> 1583
   <212> DNA
   <213> Artificial Sequence
<220>
   <223> Description of Artificial Sequence: coding sequence of the desaturase CoP construct
<220>
   <221> misc_feature
   <222> (1)..(480)
   <223> corresponding to the 5' end of the delta12-desaturase (fad2) coding region, in sense orientation
<220>
   <221> misc_feature
   <222> (1101)..(1583)
   <223> corresponding to the 5' end of the delta12-desaturase (fad2) coding region, in anti sense orientation
<400> 6
   atcattatag cctcatgctt ctactacgtc gccaccaatt acttctctct cctccctcag 60
   cctctctctt acttggcttg gccactctat tgggcctgtc aaggctgtgt cctaactggt 120
   atctgggtca tagcccacga atgcggtcac cacgcattca gcgactacca atggctggat 180
   gacacagttg gtcttatctt ccattccttc ctcctcgtcc cttacttctc ctggaagtat 240
   agtcatcgcc gtcaccattc caacactgga tccctcgaaa gagatgaagt atttgtccca 300
   aagcagaaat cagcaatcaa gtggtacggg aaatacctca acaaccctct tggacgcatc 360
   atgatgttaa ccgtccagtt tgtcctcggg tggcccttgt acttagcctt taacgtctct 420
   ggcagaccgt atgacgggtt cgcttgccat ttcttcccca acgctcccat ctacaatgac 480
   cgagaacgcc tccagatata cctctctgat gcgggtattc tagccgtctg ttttggtctt 540
   taccgttacg ctgctgcaca agggatggcc tcgatgatct gcctctacgg agtaccgctt 600
   ctgatagtga atgcgttcct cgtcttgatc acttacttgc agcacactca tccctcgttg 660
   cctcactacg attcatcaga gtgggactgg ctcaggggag ctttggctac cgtagacaga 720
   gactacggaa tcttgaacaa ggtgttccac aacattacag acacacacgt ggctcatcac 780
   ctgttctcga caatgccgca ttataacgca atggaagcta caaaggcgat aaagccaatt 840
   ctgggagact attaccagtt cgatggaaca ccgtggtatg tagcgatgta tagggaggca 900
   aaggagtgta tctatgtaga accggacagg gaaggtgaca agaaaggtgt gtactggtac 960
   aacaataagt tatgagcatg atggtgaaga aattgtcgac ctttctcttg tctgtttgtc 1020
   ttttgttaaa gaagctatgc ttcgttttaa taatcttatt gtccattttg ttgtgttatg 1080
   acattttggc tgctcattat gttcagtaac atctaccctc gcaacccctt ctttaccgtt 1140
   cgcttgggca gtatgccaga cggtctctgc aatttccgat tcatgttccc ggtgggctcc 1200
   tgtttgacct gccaattgta gtactacgca ggttctccca acaactccat aaagggcatg 1260
   gtgaactaac gactaaagac gaaaccctgt ttatgaagta gagaaagctc cctaggtcac 1320
   aaccttacca ctgccgctac tgatatgaag gtcctcttca ttccctgctc ctccttcctt 1380
   accttctatt ctggttgaca cagtaggtcg gtaaccatca gcgacttacg caccactggc 1440
   gtaagcaccc gatactgggt ctatggtcaa tcctgtgtcg gaactgtccg ggttatctca 1500
   ccggttcggt tcattctctc tccgactccc tcctctctct tcattaacca ccgctgcatc 1560
   atcttcgtac tccgatatta cta                                         1583
<210> 7
   <211> 786
   <212> DNA
   <213> Artificial Sequence
<220>
   <223> Description of Artificial Sequence: intron 2 of the Flaveria trinervia puryvate orthophosphate dikinase
<400> 7
   aagcttggta aggaaataat tattttcttt tttcctttta gtataaaata gttaagtgat 60
   gttaattagt atgattataa taatatagtt gttataattg tgaaaaaata atttataaat 120
   atattgttta cataaacaac atagtaatgt aaaaaaatat gacaagtgat gtgtaagacg 180
   aagaagataa aagttgagag taagtatatt atttttaatg aatttgatcg aacatgtaag 240
   atgatatact agcattaata tttgttttaa tcataatagt aattctagct ggtttgatga 300
   attaaatatc aatgataaaa tactatagta aaaataagaa taaataaatt aaaataatat 360
   ttttttatga ttaatagttt attatataat taaatatcta taccattact aaatatttta 420
   gtttaaaagt taataaatat tttgttagaa attccaatct gcttgtaatt tatcaataaa 480
   caaaatatta aataacaagc taaagtaaca aataatatca aactaataga aacagtaatc 540
   taatgtaaca aaacataatc taatgctaat ataacaaagc gcaagatcta tcattttata 600
   tagtattatt ttcaatcaac attcttatta atttctaaat aatacttgta gttttattaa 660
   cttctaaatg gattgactat taattaaatg aattagtcga acatgaataa acaaggtaac 720
   atgatagatc atgtcattgt gttatcattg atcttacatt tggattgatt acagttggga 780
   aagctt                                                            786